# EUROPEAN PATENT APPLICATION

(11) **EP 1 845 096 A1**
(43) Date of publication of application: **17.10.2007**
(21) Application number: 06006278.3
(22) Date of filing: 27.03.2006
(51) Int. Cl.: C07D 413/04, C07D 235/08, A61K 31/4184, A61P 35/00

(54) **12-Aza-epothilones, process for their preparation and their use as antiproliferative agents**

(71) Applicant: ETH Zürich, 8092 Zürich (CH)
(72) Inventor: Altmann, Karl-Heinz, 4153 Reinach/BL (CH); Feyen, Fabian, 8820 Wädenswill/ZH (CH); Gertsch, Jürg, 8203 Schaffhausen/SH (CH)

(57) **Abstract**

The invention relates to 12-aza-epothilones of formula (I) 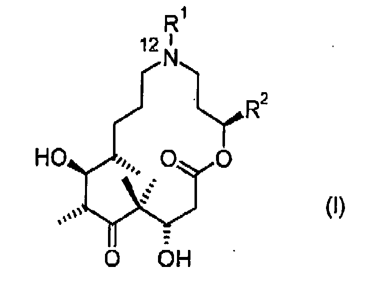 wherein R¹ is optionally substituted antinocarbonyl, optionally substituted atkoxycarbonyl, aryloxycarbonyl or heteroaryl and R² is a radical of formula (II) 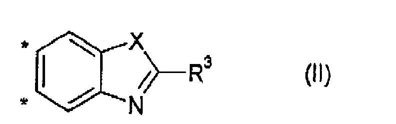 wherein X is S, O, NR⁴, -C(R⁵)=N-, -N=C(R⁵)- or -C(R⁵)=C(R⁶)-: and R³, R⁴, R⁵ and R⁶ are as defined in the specification, and which is bound to the radical of formula (I) at one of the two carbon atoms indicated with an asterix; a method of manufacture of these compounds, new intermediates, pharmaceutical preparations containing these compounds, and the use of these compounds in the treatment of proliferative diseases. These 12-aza-epothilones are highly potent cytotoxic compounds and are active against multidrug-resistant cell lines and tumours.

## Description

### Field of the invention

The invention relates to a new class of 12-aza-epothilones, the manufacture of these compounds, pharmaceutical preparations containing these compounds, and the use of these compounds in the treatment of tumours.

### Background of the invention

Epothilones A and B are natural occurring microtubule-stabilising cytotoxic agents, which inhibit the growth of human cancer cells *in vitro,* and are also active against different types of multidrug-resistant cancer cell lines. Numerous synthetic and semisynthetic analogs have been prepared and analysed (K.-H. Altmann, Current Pharm. Design 2005, 11, 1595-1613).

These epothilones have advantages over Taxol™, a branded product already introduced for the treatment of tumours, that shows the same mechanism of action but has a series of disadvantages, such as very poor water solubility, making the preparation of pharmaceutical formulations very difficult, and inefficacy on a series of tumours. Epothilones show increased water solubility and are thus more readily accessible for various pharmaceutical formulations, and are also active against muftidrug-resistant tumours (M. Wartmann and K.-H. Altmann, Curr. Med. Chem. 2002, 2, 923-148).

It is an objective of the invention to provide new epothilone analogs, which through their advantageous biological and pharmacological properties enable the armamentarium for the control of proliferative diseases such as tumours to be expanded. 12-Aza-epothilones have been described before, but these known compounds showed substantially lower activity than, for example, epothilone A (K.-H. Altmann et al., Chimia 2004, 58, 686-690). The aza-epothilones of the present invention, however, prove to be highly potent inhibitors of human tumor growth *in vitro.*

### Summary of the invention

The invention relates to 12-aza-epothilones of formula (I) 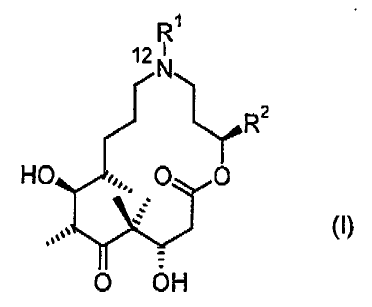 wherein R¹ is optionally substituted aminocarbonyl, optionally substituted alkoxycarbonyl, aryloxycarbonyl or heteroaryl and R² is a radical of formula (II) 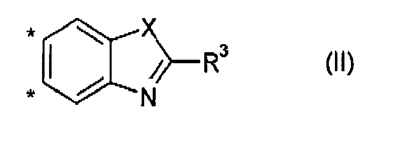 wherein X is S, O NR⁴, -C(R⁵)=N-, -N=C(R⁵)- or -C(R⁵)=C(R⁶)-; R³ is hydrogen or optionally substituted lower alkyl, R⁴ is hydrogen, optionally substituted lower alkyl or aryl; R⁵ and R⁶ are, independently of one another, hydrogen or optionally substituted lower alkyl, and which is bound to the radical of formula (I) at one of the two carbon atoms indicated with an asterix, or salts thereof;
a method of manufacture of these compounds, new intermediates, pharmaceutical preparations containing these compounds, the use of these compounds in the treatment of proliferative diseases in warm-blooded animals, such as humans, and their use in the production of pharmaceutical preparations for the treatment of proliferative diseases, e.g. tumours.

### Detailed description of the invention

Surprisingly it has been found that 12-aza-epothilones of formula (I) carrying the particular residue R² of formula (II) in position 15 have advantageous pharmaceutical properties, for example, are highly potent cytotoxic compounds and are active against multidrug-resistant cell lines and tumours.

The invention therefore relates to compounds of formula (I), wherein R¹ is optionally substituted aminocarbonyl, optionally substituted alkoxycarbonyl, aryloxycarbonyl or heteroaryl and R² is a radical of formula (II), wherein X is S, O, NR⁴, -C(R⁵)=N-, - N=C(R⁵)- or -C(R⁵)=C(R⁶)-; R³ is hydrogen or optionally substituted lower alkyl, R⁴ is hydrogen, optionally substituted lower alkyl or aryl; R⁵ and R⁶ are, independently of one another, hydrogen or optionally substituted lower alkyl, and which is bound to the radical of formula (I) at one of the two carbon atoms indicated with an asterix, or salts thereof.

The general terms used hereinbefore and hereinafter preferably have within the context of this disclosure the following meanings, unless otherwise indicated:

The prefix "lower" denotes a radical having up to and including a maximum of 7, especially up to and including a maximum of 4 carbon atoms, the radicals in question being either unbranched or branched with single or multiple branching.

Where the plural form is used for compounds, salts, and the like, this is taken to mean also a single compound, salt, or the like ("a" as an indefinite article or as a numeral meaning "one").

Asymmetric carbon atoms that are optionally present in the substituents may exist in the (R), (S) or (R,S) configuration, preferably in the (R) or (S) configuration. Substituents on a double bond or on a ring may be present in cis- (=Z-) or trans- (=E-) form. The present compounds may thus exist as mixtures of isomers or as pure isomers, preferably as pure diastereoisomers.

The radical R² of formula (II) is bonded to the radical of the molecule of formula (I) by one of the two carbon atoms marked by an asterix in formula (II). Bonding is preferably effected via the carbon atom of the benzene ring portion of R² which is in para position to X.

Alkyl is preferably an alkyl radical with 1 to 10 carbon atoms, preferably lower alkyl, especially C₁-C₄-alkyl.

Lower alkyl is unbranched or branched and is in particular methyl, ethyl or tert-butyl.

Optionally substituted lower alkyl, especially as in R³, R⁴, R⁵ and R⁶, is, for example, hydroxy-lower alkyl, halogen-lower alkyl, unsubstituted or substituted amino-lower alkyl or carbamoyl-lower alkyl, in particular hydroxy-lower alkyl or halogen-lower alkyl.

Aryl is preferably an aromatic radical with 6 to 14 carbon atoms, especially phenyl, naphthyl, fluorenyl or phenanthrenyl, whereby the said radical is unsubstituted or is substituted by one or more substituents, preferably up to three, primarily one or two substituents, especially those selected from amino; lower alkanoylamino, especially acetylamino; halogen, especially fluorine, chlorine or bromine; lower alkyl, especially methyl or also ethyl or propyl; halogen-lower alkyl, especially trifluoromethyl; hydroxy; lower alkoxy, especially methoxy or also ethoxy; phenyl-lower alkoxy, especially benzyloxy; nitro, cyano, C₈-C₁₂-alkoxy, especially n-decyloxy, carbamoyl, lower alkyl-carbamoyl, such as N-methyl- or N-tert-butylcarbamoyl, lower alkanoyl, such as acetyl, phenyloxy, halogen-lower alkyloxy, such as trifluoromethoxy or 1,1,2,2-tetrafluoroethoxy, lower alkoxycarbonyl, such as ethoxycarbonyl, lower alkylmercapto, such as methylmercapto, halogen-lower alkyl-mercapto, such as trifluoromethylmercapto, hydroxy-lower alkyl, such as hydroxymethyl or 1-hydroxymethyl, lower alkanesulphonyl, such as methanesulphonyl, halogen-lower alkane-sulphonyl, such as trifluoromethanesulphonyl, phenylsulphonyl, dihydroxybora (-B(OH)₂), 2-methyl-pyrimidin-4-yl, oxazol-5-yl, 2-methyl-1,3-dioxolan-2-yl, 1H-pyrazol-3-yl, 1-methyl-pyrazol-3-yl; and lower alkylenedioxy which is bonded to two adjacent carbon atoms, such as methylenedioxy.

In aryloxy, aryl has the meaning as described above. Aryloxy is especially phenyloxy, optionally substituted by one or more, e.g. one, two or three substituents of those mentioned above.

Heteroaryl represents an aromatic group containing at least one heteroatom selected from nitrogen, oxygen and sulfur, and is mono- or bicyclic. Monocyclic heteroaryl includes 5 or 6 membered heteroaryl groups containing 1, 2, 3 or 4 heteroatoms selected from nitrogen, sulfur and oxygen. Bicyclic heteroaryl includes 9 or 10 membered fused-ring heteroaryl groups. Examples of heteroaryl include pyrrolyl, thienyl, furyl, pyrazolyl, imidazolyl, triazolyl, oxazolyl, isoxazolyl, oxadiazolyl, thiazolyl, isothiazolyl, thiadiazolyl, pyridyl, pyridazinyl, pyrimidinyl, pyrazinyl, and benzo fused derivatives of such monocyclic heteroaryl groups, such as indolyl, benzimidazolyl, benzoxazolyl, benzoxadiazolyl, benzothiazolyl, benzothiadiazolyl, quinolinyl, isoquinolinyl, quinazolinyl, quinoxalinyl or purinyl, preferably pyrrolyl, thienyl, furyl, indolyl, benzimidazolyl, benzoxadiazolyl, benzothiazolyl, benzothiadiazolyl, pyridyl, pyrimidinyl or pyrazinyl, in particular benzoxadiazolyl or benzothiadiazolyl. The mentioned heteroaryl group may be unsubstituted or substituted. Substituents are preferably lower alkyl, halogen-lower alkyl, lower alkoxy-lower alkyl, lower alkoxy, lower alkoxy-lower alkoxy, amino optionally substituted by one or two substituents lower alkyl and one substituent lower alkylcarbonyl, halogen, or nitro.

Halogen is especially fluorine, chlorine, bromine, or iodine, in particular fluorine or chlorine.

Halogen-lower alkyl is methyl or ethyl that is substituted by halogen, such as fluorine or chlorine, especially fluoromethyl, trifluoromethyl or also chloromethyl.

Hydroxy-lower alkyl is in particular lower alkyl that is terminally substituted by hydroxy, preferably hydroxymethyl or hydroxyethyl, especially 2-hydroxyethyl.

Unsubstituted or substituted amino-lower alkyl is in particular lower alkyl that is terminally substituted by amino or substituted amino, whereby preferably one or two lower alkyl radicals are present as amino substituents, such as in aminomethyl, 2-aminoethyl, N-methylamino-methyl, 2-(N-methylamino)ethyl, N,N-dimethylaminomethyl or 2-(N,N-dimethylamino)ethyl.

Carbamoyl-lower alkyl is in particular lower alkyl that is terminally substituted by -C(O)NH₂, preferably carbamoylmethyl or 2-carbamoylethyl.

Where these radicals are present, one of radicals R³, R⁴, R⁵ and R⁶ is preferably lower alkyl, such as methyl, halogen-lower alkyl, such as fluoromethyl or trifluoromethyl, hydroxy-lower alkyl, such as hydroxymethyl or -ethyl, unsubstituted or substituted amino-lower alkyl, especially aminomethyl, 2-aminoethyl, N-methylaminomethyl, 2-(N-methylamino)ethyl, N,N-dimethylaminomethyl or 2-(N,N-dimethylamino)ethyl, and the others are hydrogen, or preferably two of the radicals R³, R⁴, R⁵ and R⁶ are lower alkyl, such as methyl, and the others are hydrogen, or preferably one of radicals R³, R⁴, R⁵ and R⁶ is lower alkyl, such as methyl, and one is hydroxy-lower alkyl, such as hydroxymethyl or - ethyl, and the others are hydrogen.

If X is NR⁴, R³ is preferably methyl, ethyl, hydroxymethyl, 2-hydroxyethyl, aminomethyl, 2-aminoethyl, N,N-dimethylaminomethyl or carbamoylmethyl, in particular hydrogen, methyl or hydroxymethyl, and R⁴ is hydrogen, methyl or ethyl.

In optionally substituted amino R¹, amino may carry one or two substituents, and these substituents are preferably lower alkyl or optionally substituted lower alkyl as listed above, for example hydroxy-lower alkyl, halogen-lower alkyl, unsubstituted or substituted amino-lower alkyl or carbamoyl-lower alkyl, in particular hydroxy-lower alkyl. Alternatively, the substituents may form a five, six or seven membered ring optionally containing oxygen, nitrogen or nitrogen substituted by lower alkyl, and corresponding substituted amino R¹ may e.g. represent pyrrolidino, piperidino, N-methylpiperazino, or morpholino.

In alkoxy, alkyl is preferably lower alkyl as described above. In optionally substituted alkoxy R¹, alkyl may be substituted by one or more, for example one, two or three, substituents selected from hydroxy, oxo, lower alkoxy, lower alkoxy-lower alkoxy, halogen, amino, lower alkylamino, di(lower alkyl)amino, lower acylamino, carboxy, carbamoyl, lower alkoxycarbonyl, cyano or phenyl. Preferably, substituted alkoxy R¹ is substituted by one substituent hydroxy, oxo, lower alkoxy, lower alkoxy-lower alkoxy, halogen, amino, lower alkylamino, di(lower alkyl)amino, lower acylamino, carboxy, carbamoyl, lower alkoxycarbonyl, cyano or phenyl, or two substituents hydroxy, lower alkoxy or halogen, or three substituents halogen. Preferably, optionally substituted alkoxy R¹ is lower alkyl, such as methyl, ethyl, isopropyl, isobutyl or tert-butyl, 2-hydroxyethyl, 2-aminoethyl, 2-methylaminoethyl, 2-dimethylaminoethyl, 2-fluoroethyl, 2,2,2-trifluoroethyl, benzyl or 2-phenylethyl.

Salts are primarily the pharmaceutically acceptable salts of compounds of formula (I).

Such salts are formed, for example, as acid addition salts, preferably with organic or inorganic acids, from compounds of formula (I) with a basic nitrogen atom, especially the pharmaceutically acceptable salts. Suitable inorganic acids are, for example, hydrohalic acids, such as hydrochloric acid, sulphuric acid, or phosphoric acid. Suitable organic acids are, for example, carboxylic, phosphonic, sulphonic or sulphamic acids, for example acetic acid, propionic acid, octanoic acid, decanoic acid, dodecanoic acid, glycolic acid, lactic acid, 2-hydroxybutyric acid, gluconic acid, glucose-monocarboxylic acid, fumaric acid, succinic acid, adipic acid, pimelic acid, suberic acid, azelaic acid, malic acid, tartaric acid, citric acid, glucaric acid, galactaric acid, amino acids, such as glutamic acid, aspartic acid, N-methylglycine, acetylaminoacetic acid, N-acetylasparagine or N-acetyloysteine, pyruvic acid, acetoacetic acid, phosphoserine, 2- or 3-glycerophosphoric acid, maleic acid, hydroxymaleic acid, methylmaleic acid, cyclohexanecarboxylic acid, benzoic acid, salicylic acid, 1- or 3-hydroxy-naphthyl-2-carboxylic acid, 3,4,5-trimethoxybenzoic acid, 2-phenoxybenzoic acid, 2-acetoxybenzoic acid, 4-aminosalicylic acid, phthalic acid, phenylacetic acid, glucuronic acid, galacturonic acid, methane- or ethane-sulphonic acid, 2-hydroxyethanesulfonic acid, ethane-1,2-disulphonic acid, benzenesulphonic acid, 2-naphthalenesulphonic acid, 1,5-naphthalene-disulphonic acid, N-cyclohexylsulphamic acid, N-methyl-, N-ethyl- or N-propyl-sulphamic acid, or other organic protonic acids, such as ascorbic acid.

For isolation or purification purposes it is also possible to use pharmaceutically unacceptable salts, for example picrates or perchlorates. Only the pharmaceutically acceptable salts or free compounds (if the occasion arises, in the form of pharmaceutical preparations) attain therapeutic use, and these are therefore preferred.

In view of the close relationship between the novel compounds in free form and in the form of their salts, including those salts that can be used as intermediates, for example In the purification or identification of the novel compounds, hereinbefore and hereinafter any reference to the free compounds is to be understood as referring also to the corresponding salts, as appropriate and expedient.

The compounds of formula I have valuable pharmacological properties, as described hereinbefore and hereinafter.

The efficacy of the compounds of formula (I) as inhibitors of microtubule depolymerisation may be proved as follows:

Tubulin is isolated as pure αβ-tubulin from fresh pig brain according to the method of Castoldi and Popov (Protein Expr. Purif. 2003, 32, 83-88). Working stock solutions of αβ-tubulin are stored at -80°C. Freshly thawed αβ-tubulin is centrifuged and then incubated with the compounds to be tested in suitable solvents and buffers, guanosine-5'-triphosphate (GTP), and glutamate in a 96-well plate, as described in more detail below in the Examples. The polymerization is monitored real-time at 340 nm in a temperature-controlled TECAN GeniosPro spectrophotometer. UV absorption of compounds is subtracted from absorption curves.

Alternatively, the degree of test-compound-induced polymerisation of microtubule protein is determined basically as described by Lin et al., Cancer Chem. Pharm. 1996, 38, 136-140: A solution of the test compound is admixed with water at room temperature. A working aliquot strain of pigs' brain microtubule protein is rapidly thawed and then diluted with suitable buffer. The polymerisation reaction is started by adding diluted microtubule protein to the test compound, followed by incubation of the sample in a water bath at room temperature. The samples are then centrifuged in order to separate polymerised from unpolymerised microtubule protein. As an indirect measure of tubulin polymerisation, the protein concentration of the supernatant (which contains the remaining non-polymerised, soluble microtubule protein) is determined by the Lowry method (CD Assay Kit, Bio-Rad Laboratories, Hercules, Calif.), and the optical density (OD) of the colour reaction is measured at 750 nm using a spectrometer. The difference in OD's between samples treated with a test compound and vehicle-treated controls is compared with that obtained with incubations containing epothilone B (positive control). The degree of polymerisation induced by a test compound is expressed relatively to the positive control (100%). By comparing the activity of several concentrations, the EC₅₀ (concentration at which 50% of the maximum polymerisation occurs) can be determined. For compounds of formula (I), the EC₅₀ lies in the range of 1 to 100, preferably in the range of 1 to 50, especially from 1 to 10 µM.

The efficacy against tumour cells may be demonstrated in the following way:

Stock solutions of the test compounds (2 mM) are prepared in DMSO and stored at -20°C. Human KB-31 and (multidrug-resistant, P-gp170 expressing) KB-8511 epidermoid carcinoma cells (Akiyama et al., Somat. Cell. Mol. Genetics 1985, 11, 117-120), which are both derivatives of the KB cell line (ATCC) and are human epidermis carcinoma cells, are employed. KB 31 cells may be cultivated in mono-layers using Dulbecco's modified Eagle's medium (D-MEM) with 10% foetal calf serum (M.A. Bioproducts), L-glutamine (Flow), penicillin (50 units/ml) and streptomycin (50 µg/ml) (Flow); they then grow at a duplication time of ca. 22 hours, and their relative plating efficiency is ca. 60%. KB-8511 is a variant derived from the KB-31 cell line, which is obtained using colchicine treatment cycles, and has an approximately 40 times relative resistance to colchicine compared with KB-31 cells. The cells are incubated at 37°C in an incubator with 5% v/v CO₂ and at 80% relative humidity with MEMAlpha medium which contains ribonucleosides and desoxyribonucleosides (Gibco BRL), complemented with 10 IU penicillin, 10 µg/ml streptomycin and 5% foetal calf serum. The cells are seeded in a quantity of 1.5 x 10³ cells/well in 96-well microtitre plates, and incubated over night. Serial dilutions of the test compounds in culture medium are added on day 1. The plates are then incubated for a further 4 days, after which the cells are fixed with 3.3% v/v glutaraldehyde, washed with water and dyed with 0.05% w/v methylene blue. After washing, the dye is eluted with 3% HCl and the optical density measured at 665 nm with a GeniosPro plate spectrophotometer (Tecan, Switzerland). IC₅₀ values are determined by adaptation of mathematical curves, using the formula [(OD treated)-(OD start) [/] (OD control)-(OD start)] x 100. The IC₅₀ is defined as the concentration of a test compound at the end of the incubation period, which led to 50% of the cell count per well compared with the control (concentration at semi-maximum inhibition of cell growth). Compounds of formula (I) thus preferably show an IC₅₀ in the range of 0.1 x 10⁻⁹ to 500 x 10⁻⁹ M, preferably between 0.2 and 50 nM.

Tests on other tumour cells lines can also be carried out in a comparable manner. The preferred ranges of the IC₅₀ values of compounds of formula (I) are given in square parentheses. A549 (lung; ATCC CCL 185) [0.01 to 100 nM], HCT-116 (colon) [0.05 to 200 nM], and PC-3M cells (prostate, hormone-insensitive derivative derived from PC-3, ATCC CRL 1435) [0.05 to 500 nM] were grown in RPMI-1640 medium supplemented with 10% FBS.

The *in vivo* efficacy may be demonstrated as follows: The models used are xenotransplants of tumours, such as KB-31 or KB-8511 epidermoid tumours, in mice. The anti-tumour efficacy of the test compounds may be measured in female BLB/c nu/nu mice, for example against the corresponding subcutaneously transplanted cell line. To this end, tumour fragments of about 25 mg are implanted into the left side of each of the mice (for example 6 animals per dose). The test compound is administered for example on day 11 after transplantation in different dosages (for example 0.1; 0.5; 1; 5 and 10 mg/kg), if desired repeating the administration, if required several times, after between two days and two weeks. The volumes of the tumours are determined for example after about 2 to 4 weeks (e.g. two weeks after the start of treatment). The tumour volumes are calculated by measuring the tumour diameter along two vertically arranged axes and according to published methods (see Evans et al., Brit. J. Cancer 1982, 45, 466-8). The anti-tumour efficacy is determined as the mean increase in tumour volume of the treated animals divided by the mean increase in tumour volume of the untreated animals (controls) and, after multiplication by 100, is expressed as T/C %. Tumour regression (given in %) is calculated as the smallest mean tumour volume (Vt) in relation to the mean tumour volume at the start of treatment (Vo) according to the formula % regression = [1 -(Vt/Vo)] x 100. In this case also, other cell lines can be used, for example those named above in the demonstration of efficacy against tumour cells.

Values for the in vitro activity of four compounds of the invention **(1, 2, 3, 17)** and reference compounds **A** (unsaturated in position 9/10), **B** (12-aza-epothilone with a different substituent R², see K.-H. Altmann et al., Chimia 2004, 58, 686-690) and C (Epothilone A) are collected in Table 1.

**Table 1: Tubulin-polymerizing and antiproliferative activity of compounds 1, 2, 3 and 17 and reference compounds A, B and C**

| Compound | EC₅₀ Tubulin polymerization [µM]^{[a]} | IC₅₀ [nM]^{[b]} | | | | |
|---|---|---|---|---|---|---|
| | | A549 | HCT-116 | PC-3M | KB-31 | KB-8511 |
| **1** | 3.9 ± 0.6 | 1.9 ± 0.4 | 1.6 ± 0.5 | 2.3 ± 0.6 | 0.35 | 222 |
| **2** | 5.4 ± 0.4 | 18 ± 3.1 | 23.9 ± 2.9 | 12.3 ± 1.1 | | |
| **3** | 5.1 ± 0.5 | 11.5 ± 1.8 | 17.1 ± 3.3 | 6.3 ± 2.1 | | |
| **17** | 7.2 ± 0.6 | 329 ± 237 | 206 ± 8 | 244 ± 45 | | |
| **A** | 9.1 ± 0.7 | 920 ± 85 | 1009 ± 71 | 973 ± 64 | | |
| **B** | 5.6 ± 0.4 | 130 ± 24 | 110 ± 19 | 126 ± 22 | 31 | 105 |
| **C** | 4.6 ± 0.5 | 3.2 ± 0.5 | 2.2 ± 0.3 | 3.4 ± 0.4 | | |

| | | | | | | |
|---|---|---|---|---|---|---|
| ^{[a]} Concentration required to induce 50% of the maximum αβ-tubulin polymerization achievable with the respective compound (10 µM of porcine brain αβ-tubulin). Tubulin polymerization was determined through turbidity measurements at 340 nm (A₃₄₀). For a given compound concentration the achievement of an equilibrium state between soluble and polymerized tubulin is indicated by a stable plateau in A₃₄₀. Maximum tubulin polymerization is reached when increases in compound concentration no longer result in an increase of the plateau value for A₃₄₀. Similar maximum values for A₃₄₀ were observed for all compounds investigated in this study except for A. ^{[b]} IC₅₀ values for human cancer cell growth inhibition. KB-31, KB-8511: cervical (or cervix); A549: lung; HCT-116: colon; PC-3M: prostate. KB-8511 is a P-glycoprotein 170 (P-gp170) overexpressing multidrug-resistant subline of the KB-31 parental line. Cells were exposed to compounds for 72 h. Cell numbers were determined by quantification of protein content of fixed cells by methylene blue staining. Values represent the means of at least three independent experiments (± standard deviation). | | | | | | |

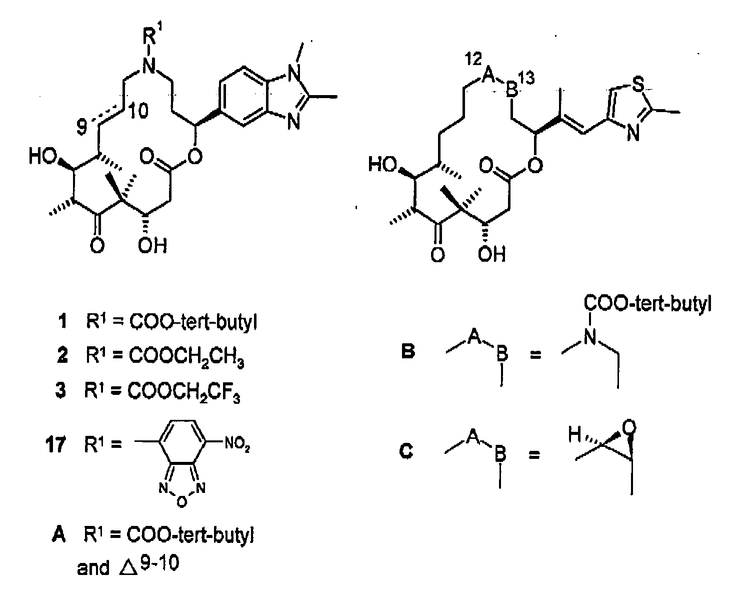

The antiproliferative activity of **1** is thus comparable with that of Epothilone A **(C),** with the exception of the multidrug-resistant KB-8511 line, where **1** is significantly less potent. Likewise, **1** induces tubulin polymerization *in vitro* with similar potency to Epothilone A (**C**), which strongly suggests that the inhibition of human cancer cell proliferation by **1,** as for natural epothilones, is a consequence of interference with microtubule functionality.

From Table 1 it is apparent that **1** is > 60-fold more potent against drug-sensitive human cancer cells than the corresponding parent (natural side-chain-containing) aza-epothilone **B.** It should be noted that the potency increase observed for **1** over **B** dramatically exceeds the potency-enhancing effects previously observed for the dimethyl-benzimidazole side chain in combination with polyketide-based macrocycles (2 to 15-fold, see K.-H. Altmann et al., Bioorg. Med. Chem. Lett. 2000, 10, 2765-2768). Equally intriguing is the observation that compound **A**, which incorporates a *trans* double bond between C9 and C10, is significantly less potent than the fully saturated aza-epothilone 1, both at the levels of tubulin polymerization as well as cellular activity. These findings are in marked contrast to the effects observed for Epothilones B and D, where the introduction of a *trans* double bond between C9 and C10 results in enhanced cellular potency (A. Rivkin et al., J. Am. Chem. Soc. 2004, 126, 10913-10922).

Compared to aza-epothilone **1,** analogs **2** and **3** exhibit somewhat reduced cellular activity (Table 1), but both compounds still remain very potent antiproliferative agents against all drug-sensitive cancer cell lines investigated. Based on the tubulin polymerization data summarized in Table **1,** little difference seems to exist between the three compounds in their ability to interfere with microtubule functionality, thus suggesting that the observed differences in cellular potency are also caused by non-target related parameters such as cellular uptake or intracellular distribution. Compound **17** is clearly less active.

Owing to these properties, the compounds of the invention are suitable for the treatment of proliferative diseases, especially tumour diseases, including metastases; for example solid tumours such as lung tumours, breast tumours, colorectal tumours, prostate tumours, melanomas, brain tumours, pancreas tumours, neck tumours, bladder tumours, neuroblastomas, throat tumours, but also proliferative diseases of blood cells, such as leukaemia; also for the treatment of other diseases which respond to treatment with microtubule depolymerisation inhibitors, such as psoriasis.

A compound of formula (I) can be administered alone or in combination with one or more other therapeutic agents; possible combination therapy taking the form of fixed combinations or the administration of a compound of the invention and one or more other therapeutic agents being staggered or given independently of one another, or the combined administration of fixed combinations and one or more other therapeutic agents. A compound of formula (I) can besides or in addition be administered for tumour therapy in combination with chemotherapy, radiotherapy, immunotherapy, surgical intervention, or a combination of these. Long-term therapy is equally possible as is adjuvant therapy in the context of other treatment strategies, as described above. Other possible treatments are therapy to maintain the patient's status after tumour regression, or even chemopreventive therapy, for example in patients at risk.

Therapeutic agents for possible combination are especially one or more anti proliferative, cytostatic or cytotoxic compounds, for example one or more chemotherapeutic agent(s) selected from the group comprising the classical chemotherapeutic agents, an inhibitor of polyamine biosynthesis, an inhibitor of protein kinases, especially of serine/threonine protein kinases, such as protein kinase C, or of tyrosine protein kinases, such as epidermal growth factor receptor protein tyrosine kinase, a cytokine, a negative growth regulator, such as TGF-β or IFN-β, an aromatase inhibitor, and a classical cytostatic.

Compounds according to the invention are not only for the (prophylactic and preferably therapeutic) treatment of humans, but also for the treatment of other warm-blooded animals, for example of commercially useful animals, for example rodents, such as mice, rabbits or rats, or guinea-pigs. They may also be used as a reference standard in the test systems described above to permit a comparison with other compounds.

A compound of formula (I) may also be used for diagnostic purposes, for example with tumours that have been obtained from warm-blooded animal "hosts", especially humans, and implanted into mice to test them for decreases in growth after treatment with such a compound, in order to investigate their sensitivity to the said compound and thus to improve the detection and determination of possible therapeutic methods for neoplastic diseases in the original host.

Within the groups of preferred compounds of formula (I) mentioned hereinafter, definitions of substituents from the general definitions mentioned hereinbefore may reasonably be used, for example, to replace more general definitions with more specific definitions or especially with definitions characterized as being preferred; the definitions characterised as being preferred, or exemplary ("e.g.", "such as", "for example") are preferred.

Preference is given to a compound of formula (I), wherein
R¹ is aminocarbonyl, lower alkylaminocarbonyl, di-lower alkylaminocarbonyl, hydroxy-lower alkylaminocarbonyl, halogen-lower alkylaminocarbonyl, amino-lower alkylaminocarbonyl, lower alkylamino-lower alkylaminocarbonyl, di-lower alkylamino-lower alkylaminocarbonyl, carbamoyl-lower alkylaminocarbonyl; substituted aminocarbonyl wherein the substituents on nitrogen form a five, six or seven membered ring optionally containing oxygen, nitrogen or nitrogen substituted by lower alkyl; alkoxycarbonyl, hydroxy-lower alkoxycarbonyl, oxo-lower alkoxycarbonyl, lower alkoxy-lower alkoxycarbonyl, lower alkoxy-lower alkoxy-lower alkoxycarbonyl, halogen-lower alkoxycarbonyl, amino-lower alkoxycarbonyl, lower alkylamino-lower alkoxycarbonyl, di(lower alkyl)amino-lower alkoxycarbonyl, lower acylamino-lower alkoxycarbonyl, carboxy-fower alkoxycarbonyl, carbamoyl-lower alkoxycarbonyl, lower alkoxycarbonyl-lower alkoxycarbonyl, cyano-lower alkoxycarbonyl, phenyl-lower alkoxycarbonyl, di-hydroxy-lower alkoxycarbonyl, di-lower alkoxy-lower alkoxycarbonyl, di-halo-lower alkoxycarbonyl, tri-halo-lower alkoxycarbonyl; optionally substituted phenyloxycarbonyl, wherein the substituents are selected from amino; lower alkanoylamino, halogen, lower alkyl, halogen-lower alkyl, hydroxy; lower alkoxy, phenyl-lower alkoxy, nitro, cyano, C₈-C₁₂-alkoxy, carbamoyl, lower alkyl-carbamoyl, lower alkanoyl, phenyloxy, halogen-lower alkyloxy, lower alkoxycarbonyl, lower alkylmercapto, halogen-lower alkyl-mercapto, hydroxy-lower alkyl, lower alkanesulphonyl, halogen-lower alkane-sulphonyl, phenylsulphonyl, dihydroxyboranyl, 2-methyl-pyrimidin-4-yl, oxazol-5-yl, 2-methyl-1,3-dioxolan-2-yl, 1H-pyrazol-3-yl, 1-methyl-pyrazol-3-yl, and lower alkylenedioxy which is bonded to two adjacent carbon atoms; or pyrrolyl, thienyl, furyl, pyrazolyl, imidazolyl, triazolyl, oxazolyl, isoxazolyl, oxadiazolyl, thiazolyl, isothiazolyl, thiadiazolyl, pyridyl, pyridazinyl, pyrimidinyl, pyrazinyl, indolyl, benzimidazolyl, benzoxazolyl, benzoxadiazolyl, benzothiazolyl, benzothiadiazolyl, quinolinyl, isoquinolinyl, quinazolinyl, quinoxalinyl or purinyl, each optionally substituted by lower alkyl, halogen-lower alkyl, lower alkoxy-lower alkyl, lower alkoxy, lower alkoxy-lower alkoxy, amino optionally substituted by one or two substituents lower alkyl and one substituent lower alkylcarbonyl, halogen, or nitro; and
R² is a radical of formula (II), which is bound to the radical of formula (I) at one of the two carbon atoms indicated with an asterix, preferably by the carbon atom in para-position to X; wherein
R³ is hydrogen, lower alkyl, hydroxy-lower alkyl, halogen-lower alkyl, amino-lower alkyl, lower alkyl-amino-lower alkyl, di-lower alkyl-amino-lower alkyl or carbamoyl-lower alkyl;
X is S, O NR⁴, -C(R⁵)=N-, -N=C(R⁵)- or -C(R⁶)=C(R⁶)-;
R⁴ is hydrogen, lower alkyl, hydroxy-lower alkyl, halogen-lower alkyl, amino-lower alkyl, lower alkyl-amino-lower alkyl, di-lower alkyl-amino-lower alkyl, carbamoyl-lower alkyl, or phenyl; and
R⁵ and R⁶ are, independently of one another, hydrogen, lower alkyl, hydroxy-lower alkyl, halogen-lower alkyl, amino-lower alkyl, lower alkyl-amino-lower alkyl, di-lower alkyl-amino-lower alkyl or carbamoyl-lower alkyl;
or salts thereof.

More preferred is a compound of formula (I), wherein
R¹ is aminocarbonyl, lower alkylaminocarbonyl, di-lower alkylaminocarbonyl, hydroxy-lower alkylaminocarbonyl, pyrrolidinocarbonyl, piperidinocarbonyl, N-methylpiperazinocarbonyl, morpholinocarbonyl; lower alkoxycarbonyl, hydroxy-lower alkoxycarbonyl, lower alkoxy-lower alkoxycarbonyl, lower alkoxy-lower alkoxy-lower alkoxycarbonyl, halogen-lower alkoxycarbonyl, phonyl-lower alkoxycarbonyl, di-hydroxy-lower alkoxycarbonyl, di-lower alkoxy-lower alkoxycarbonyl, di-halo-lower alkoxycarbonyl, tri-halo-lower alkoxycarbonyl; optionally substituted phenyloxycarbonyl, wherein the substituents are selected from amino; lower alkanoylamino, halogen, lower alkyl, halogen-lower alkyl, hydroxy; lower alkoxy, lower alkanoyl, and lower alkylenedioxy which is bonded to two adjacent carbon atoms; or pyrrolyl, thienyl, furyl, indolyl, benzimidazolyl, benzoxadiazolyl, benzothiazolyl, benzothiadiazolyl, pyridyl, pyrimidinyl or pyrazinyl, each optionally substituted by lower alkyl, halogen-lower alkyl, lower alkoxy-lower alkyl, lower alkoxy, lower alkoxy-lower alkoxy, amino optionally substituted by one or two substituents lower alkyl and one substituent lower alkylcarbonyl, halogen, or nitro; and
R² is a radical of formula (II), which is bound to the radical of formula (I) at one of the two carbon atoms indicated with an asterix, preferably by the carbon atom in para-position to X; wherein
R³ is hydrogen, lower alkyl, hydroxy-lower alkyl, halogen-lower alkyl, amino-lower alkyl, lower alkyl-amino-lower alkyl, di-lower alkyl-amino-lower alkyl or carbamoyl-lower alkyl;
X is S, O, NR⁴, -C(R⁵)=N-, -N=C(R⁵)- or-C(R⁵)=C(R⁶)-;
R⁴ is hydrogen, lower alkyl, hydroxy-lower alkyl, halogen-lower alkyl, amino-tower alkyl, lower alkyl-amino-lower alkyl, di-lower alkyl-amino-lower alkyl or carbamoyl-lower alkyl; and
R⁵ and R⁶ are, independently of one another, hydrogen, lower alkyl, hydroxy-lower alkyl, or halogen-lower alkyl;
or salts thereof.

Further preferred is a compound of formula (I), wherein
R¹ is aminocarbonyl, lower alkylaminocarbonyl, di-lower alkylaminocarbonyl, hydroxy-lower alkylaminocarbonyl; lower alkoxycarbonyl, hydroxy-lower alkoxycarbonyl, lower alkoxy-lower alkoxycarbonyl, halogen-lower alkoxycarbonyl, phenyl-lower alkoxycarbonyl, di-halo-lower alkoxycarbonyl, tri-halo-lower alkoxycarbonyl, phenyloxycarbonyl; or benzoxadiazolyl or benzothiadiazolyl, each optionally substituted by lower alkyl, halogen-lower alkyl, halogen, or nitro; and
R² is a radical of formula (II), which is bound to the radical of formula (I) at one of the two carbon atoms indicated with an asterix, preferably by the carbon atom in para-position to X; wherein
R³ is hydrogen, lower alkyl, hydroxy-lower alkyl, halogen-lower alkyl, amino-lower alkyl, lower alkyl-amino-lower alkyl, di-lower alkyl-amino-lower alkyl or carbamoyl-lower alkyl;
X is S, O, NR⁴, -C(R⁵)=N-, -N=C(R⁵)- or -C(R⁵)=C(R⁶)-;
R⁴ is hydrogen, lower alkyl, hydroxy-lower alkyl, halogen-lower alkyl, amino-lower alkyl, lower alkyl-amino-lower alkyl, di-lower alkyl-amino-lower alkyl or carbamoyl-lower alkyl; and
R⁵ and R⁶ are, independently of one another, hydrogen, lower alkyl, hydroxy-lower alkyl, or halogen-lower alkyl;
or salts thereof.

Special preference is given to a compound of formula (I), wherein
R¹ is aminocarbonyl, lower alkylaminocarbonyl, di-lower alkylaminocarbonyl, hydroxy-lower alkylaminocarbonyl; lower alkoxycarbonyl, hydroxy-lower alkoxycarbonyl, lower alkoxy-lower alkoxycarbonyl, halogen-lower alkoxycarbonyl, phenyl-lower alkoxycarbonyl, di-halo-lower alkoxycarbonyl, tri-halo-lower alkoxycarbonyl, phenyloxycarbonyl; or benzoxadiazolyl or benzothiadiazolyl, each optionally substituted by lower alkyl, halogen-lower alkyl, halogen, or nitro; and
R² is a radical of formula (II), which is bound to the radical of formula (I) at one of the two carbon atoms indicated with an asterix, preferably by the carbon atom in para-position to X; wherein
R³ is hydrogen, methyl, ethyl, isopropyl, hydroxymethyl, 2-hydroxyethyl, aminomethyl, 2-aminoethyl, 2-dimethylaminoethyl, carbamoylmethyl, fluoromethyl, fluoroethyl, trifluoromethyl or 2,2,2-trifluoroethyl;
X is S, O NR⁴, wherein R⁴ is hydrogen, methyl, 2-hydroxyethyl, 2-aminoethyl, 2-dimethylaminoethyl or carbamoylmethyl; -C(R⁵)=N- or -N=C(R⁵)-, wherein R⁵ is hydrogen, methyl, hydroxymethyl, 2-hydroxyethyl, fluoromethyl, or trifluoromethyl, or -C(R⁵)=C(R⁶)-, wherein one of R⁶ or R⁶ is hydrogen and the other one is hydrogen, methyl, hydroxymethyl, 2-hydroxyethyl, fluoromethyl, or trifluoromethyl;
or salts thereof.

Further special preference is given to a compound of formula (I), wherein
R¹ is lower alkoxycarbonyl, hydroxy-lower alkoxycarbonyl, lower alkoxy-lower alkoxycarbonyl, halogen-lower alkoxycarbonyl, phenyl-lower alkoxycarbonyl, di-halo-lower alkoxycarbonyl, tri-halo-lower alkoxycarbonyl, or benzoxadiazolyl optionally substituted by lower alkyl, halogen-lower alkyl, halogen, or nitro; and
R² is a radical of formula (II), which is bound to the radical of formula (I) at one of the two carbon atoms indicated with an asterix, preferably by the carbon atom in para-position to X; wherein
R³ is hydrogen, methyl, ethyl, hydroxymethyl, 2-hydroxyethyl, fluoromethyl, or trifluoromethyl;
X is S, O NR⁴, wherein R⁴ is hydrogen, methyl or 2-hydroxyethyl; -C(R⁵)=N- or - N=C(R⁵)-, wherein R⁵ is hydrogen, methyl, hydroxymethyl, 2-hydroxyethyl, fluoromethyl, or trifluoromethyl; or -C(R⁵)=C(R⁶)-, wherein one of R⁵ or R⁶ is hydrogen and the other one is hydrogen, methyl, hydroxymethyl, 2-hydroxyethyl, fluoromethyl, or trifluoromethyl; or salts thereof.

Particular preference is given to a compound of formula (I), wherein
R¹ is lower alkoxycarbonyl, phenyl-lower alkoxycarbonyl, benzoxadiazolyl or nitro-benzoxadiazolyl; and
R² is a radical of formula (II), which is bound to the radical of formula (I) at one of the two carbon atoms indicated with an asterix, preferably by the carbon atom in para-position to X; wherein
R³ is hydrogen, methyl, ethyl, hydroxymethyl, 2-hydroxyethyl, fluoromethyl, or trifluoromethyl;
X is S, NR⁴, wherein R⁴ is hydrogen or methyl, or -C(R⁵)=C(R⁶)-, wherein R⁵ and R⁶ are hydrogen;
or salts thereof.

Of the compounds and (where appropriate) groups including the compounds, as mentioned hereinbefore and hereinafter, the following are preferred in particular (the free compounds being understood to mean also the corresponding salts):
(a) compounds of formula (I), wherein X is S;
(b) compounds of formula (I), wherein X is NH;
(c) compounds of formula (I), wherein X is NCH₃;
(d) compounds of formula (I), wherein X is -CH=CH-;
(e) compounds of formula (I), wherein R¹ is C₁-C₄-alkoxycarbonyl [also if they fall within one of the definitions (a) to (d)]; or
(f) compounds of formula (I), wherein R¹ is 7-nitro-4-benzoxadiazolyl [also if they fall within one of the definitions (a) to (d)];

In the compounds named under the above definitions (a) to (f), the remaining radicals respectively have the meanings given hereinbefore and hereinafter for compounds of formula (I), especially those characterised as being preferred meanings. Of the compounds of formula (I) and their salts falling within definitions (a) to (f), particular preference is given to those in which the radical R² of formula (II) is linked by the bond in para position to X, i.e. is a radical of formula (IIa), 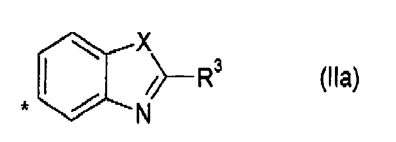 wherein the asterix denotes the position connecting the radical of formula (IIa) to the radical of formula (I).

Particularly preferred are compounds of formula (I) wherein the radical R² is of formula

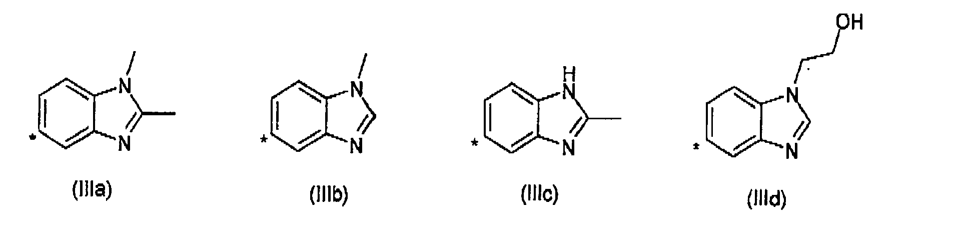

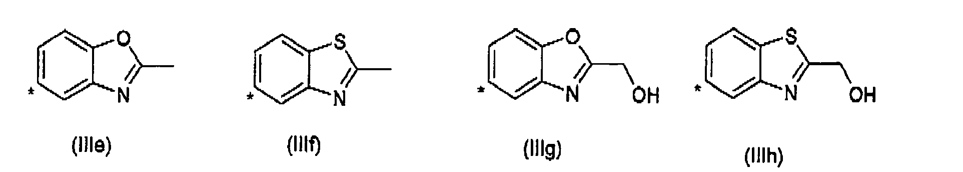

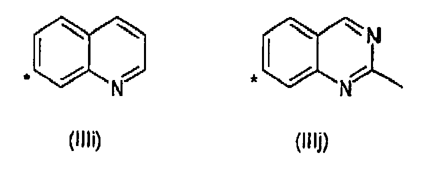

Particularly preferred are compounds of formula (I) wherein the radical R² is of formula (IIIa)

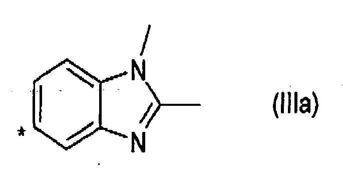

Especially preferred are the compounds named in the examples, or salts thereof (especially pharmaceutically acceptable salts), provided that a salt-forming group is present.

The compounds of formula (I) may be prepared by methods known per se, preferably in that
a) a diene of formula (IV) 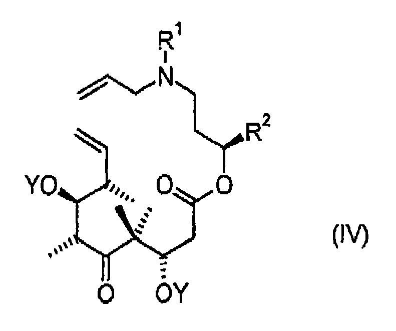 wherein R¹ and R² have the meanings given for compounds of formula (I), Y is a protecting group and wherein further functional groups that should not participate in the reaction are present if necessary in protected form, is cyclised in a metathesis reaction, the resulting double bond in position 9/10 is hydrogenated, and protecting groups Y and any further protecting group are removed, or
b) a hydroxy-acid of formula (V) 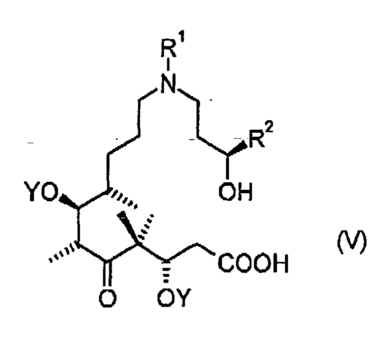
wherein R¹ and R² have the meanings given for compounds of formula (I), Y is a protecting group and wherein further functional groups that should not participate in the reaction are present if necessary in protected form, is cyclised, and protecting groups Y and any further protecting group are removed,
and, if desired, an obtainable compound of formula (I) is converted into a different compound of formula (I); an obtainable free compound of formula (I) is converted into a salt; an obtainable salt of a compound of formula (I) is converted into another salt or the free compound of formula (I); and/or obtainable isomeric mixtures of compounds of formula (I) are separated into the individual isomers.

If one or more other functional groups, for example hydroxy, carboxy or amino, in a compound of formula (IV) or (V) are or need to be protected, because they should not take part in the reaction, these are those usually used in the synthesis of peptide compounds, and also of cephalosporins and penicillins, as well as nucleic acid derivatives and sugars. Such protecting groups Y are also chosen to protect the hydroxy functions in position 3 and 7 in the form of a substituent OY. The protecting groups may already be present in precursors and should protect the functional groups concerned against unwanted secondary reactions, such as acylations, etherifications, esterifications, oxidations, reduction, solvolysis, and similar reactions. In certain cases, the protecting groups may, in addition to this protection, effect a selective, for example stereoselective, course of reactions. It is a characteristic of protecting groups that they lend themselves readily, i.e. without undesired secondary reactions, to removal, typically by solvolysis, reduction, photolysis or also by enzyme activity, for example under conditions analogous to physiological conditions, and that they are not present in the end products. The specialist knows, or can easily establish, which protecting groups are suitable for the reactions mentioned hereinabove and hereinafter.

The protection of functional groups by such protecting groups, the protecting groups themselves, and their removal reactions are described for example in standard reference works, such as J. F. W. McOmie, "Protective Groups in Organic Chemistry", Plenum Press, London and New York 1991, or in T.W. Greene and P.G.M. Wuts, "Protective Groups in Organic Synthesis", Wiley, 3rd edition 1999.

In reaction a) cyclisation is accomplished by a metathesis reaction providing a olefinic macrocycle corresponding to a compound of formula (I) with a double bond in 9/10 position, and ethylene. Such metathesis reaction is performed under standard conditions, preferably using ruthenium catalysts, e.g. Grubb's catalysts, in particular second generation Grubb's catalysts based on dihydroimidazol-2-ylidene ruthenium. The obtained olefinic macrocycle is then reduced to a compound of formula (I) by any of the standard hydrogenation reaction, preferably by diimide reduction.

In reaction b) cyclisation may be effected under conventional conditions of macrolactonisation. The lactonisation of a compound of formula (V) takes place in the presence of a coupling reagent, for example a compound that can convert the free acid of formula (V) into an activated form, for example by forming an anhydride, an acid halide, or an activated amide, especially by a reaction with an activating reagent used in peptide chemistry, and if necessary in the presence of an inert base, possibly a tertiary nitrogen base, e.g. triethylamine or N,N-dimethylaminopyridine, at preferred temperatures of between -10 and 100°C, preferably between 0 and 75°C. The reaction may also be carried out in such a way that first of all an activated form of the acid of formula (V) is produced, for example an anhydride or activated amide, and then this anhydride or amide is cyclised to the corresponding lactone, whereby both reactions can also follow immediately one another in one and the same reaction mixture. The reactions are preferably effected in suitable solvents or solvent mixtures, such as ethers, e.g. tetrahydrofuran, aromatic hydrocarbons such as toluene, or dipolar aprotic solvents, e.g. dimethylformamide.

In the additional process steps, carried out as desired, functional groups of the starting compounds which should not take part in the reaction may be present in unprotected form or may be protected for example by one or more of the protecting groups mentioned hereinabove. The protecting groups are then wholly or partly removed according to standard methods.

For example, compounds of formula (I) wherein R¹ is tert-butoxycarbonyl may be treated with a suitable acid known to cleave a tert-butoxycarbonyl protecting group, e.g. trifluoroacetic acid or pyridinium hydrofluoride, and then the resulting secondary amine acylated (or heteroarylated) with a suitable reagent introducing another radical R¹. Such reagents may be an acid halide, acid anhydride, activated amide, or halogenated heteroaryl group.

Salts of compounds of formula (I) with a salt-forming group may be prepared in a manner known per se. Acid addition salts of compounds of formula I may thus be obtained e.g. by treatment with an acid or with a suitable anion exchange reagent.

Salts can usually be converted to free compounds, e.g. by treating with suitable basic agents, for example with alkali metal carbonates, -hydrogencarbonates, or -hydroxides, typically potassium carbonate or sodium hydroxide.

Stereoisomeric mixtures, e.g. mixtures of diastereoisomers, can be separated into their corresponding isomers in a manner known per se by means of suitable separation methods. Diastereoisomeric mixtures may thus be separated into their individual diastereoisomers by means of fractionated crystallization, chromatography, solvent distribution, and similar procedures. This separation may take place either at the stage of one of the starting compounds or in a compound of formula (I) itself. Enantiomers may be separated through the formation of diastereoisomeric salts, for example by salt formation with an enantiomer-pure chiral acid, or by means of chromatography, for example by HPLC, using chromatographic substrates with chiral ligands. Enantiomer separation is normally effected at the intermediate stage.

The starting materials of formula (IV) and (V) may likewise be produced by known processes, for example as described in the following, in particular as described in the Examples in more detail, or in analogy to these reactions.

Compounds of formula (IV) are formed by condensation reaction of a compounds of formula (VI) with a compound of formula (VII) under standard conditions for ester formation. Preferably, functional groups other than those involved in ester formation are in protected form, e.g. as shown for the hydroxy functions in a compound of formula (VI).

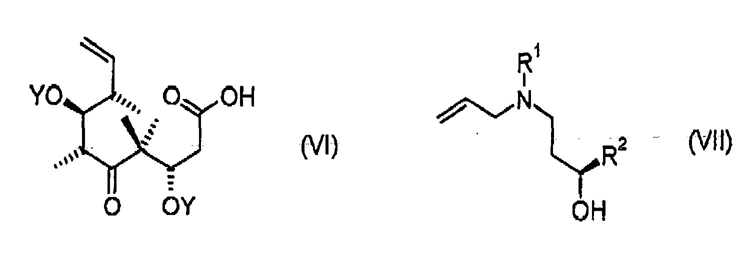

Compounds of formula (V) may be formed, for example, by a series of reactions known per se in the art, starting with condensation of an amine of formula (VIII) with an aldehyde of formula (IX) under reductive conditions (reductive amination), acylation of the obtained secondary amine introducing a residue of formula R¹, and selective cleavage of the protecting group Y¹ followed by oxidation of the hydroxy function to the carboxy group. Preferably, functional groups other than those involved in the reaction steps are in protected form, e.g. as shown for the hydroxy functions in the compounds of formula (VIII) and (IX).

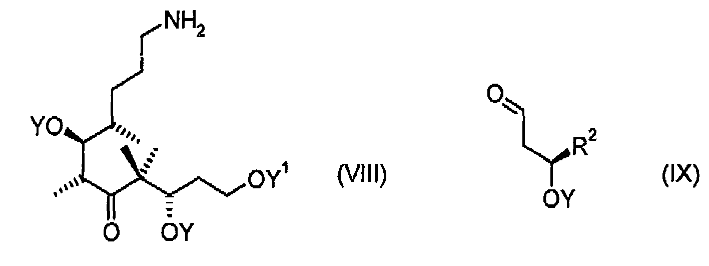

All process steps described here can be carried out under known reaction conditions, preferably under those specifically mentioned, in the absence of or usually in the presence of solvents or diluents, preferably those that are inert to the reagents used and able to dissolve them, in the absence or presence of catalysts, condensing agents or neutralising agents, for example ion exchangers, typically cation exchangers, for example in the H+ form, depending on the type of reaction and/or reactants at reduced, normal, or elevated temperature, for example in the range from -100°C to about 190°C, preferably from about -80°C to about 150°C, for example at -80 to -60°C, at room temperature, at -20 to 40°C or at the boiling point of the solvent used, under atmospheric pressure or in a closed vessel, if required under pressure, and/or in an inert, for example an argon or nitrogen, atmosphere.

Salts may be present in all starting compounds and intermediates, if these contain salt-forming groups. Salts may also be present during the reaction of such compounds, provided that the reaction is not thereby disturbed.

At all reaction stages, isomeric mixtures that occur can be separated into their individual isomers, e.g. diastereoisomers or enantiomers, or into any mixtures of isomers,.e.g. racemates or diastereoisomeric mixtures, for example analogously to methods described above.

The solvents from which those can be selected which are suitable for the reaction In question include for example water, esters, such as lower alkyl-lower alkanoate, e.g ethyl acetate, ethers, such as aliphatic ethers, e.g. diethylether, or cyclic ethers, e.g. tetrahydrofuran, liquid aromatic hydrocarbons, such as benzene or toluene, alcohols, such as methanol, ethanol or 1- or 2-propanol, nitriles, such as acetonitrile, halogenated hydrocarbons, such as methylene chloride, acid amides, such as dimethylformamide, bases, such as heterocyclic nitrogen bases, e.g. pyridine, carboxylic acids, such as lower alkanecarboxylic acids, e.g. acetic acid, carboxylic acid anhydrides, such as lower alkane acid anhydrides, e.g. acetic anhydride, cyclic, linear, or branched hydrocarbons, such as cyclohexane, hexane, or isopentane, or mixtures of these solvents, e,g- aqueous solutions, unless otherwise stated in the description of the process. Such solvent mixtures may also be used in working up, for example by chromatography or partitioning.

The invention relates also to those embodiments of the process in which one starts from a compound obtainable at any stage as an intermediate and carries out the missing steps, or breaks off the process at any stage, or forms a starting material under the reaction conditions, or uses said starting material in the form of a reactive derivative or salt, or produces a compound obtainable by means of the process according to the invention under the process conditions therein, and further processes the said compound in situ. In the preferred embodiment, one starts from those starting materials which lead to the compounds described hereinabove as preferred, particularly as especially preferred, primarily preferred, and/or preferred above all.

In particular, the invention also relates to intermediate products of formulae (IV), (V), (VI), (VII), (VIII) and (IX), and corresponding intermediates wherein OY and/or OY¹ represent OH, and wherein the radicals are respectively defined as mentioned, and especially have the preferred meanings for compounds of formula (I).

In the preferred embodiment, compounds of formula (I) are prepared analogously to the processes and process steps defined in the examples.

The compounds of formula (I), including their salts, are also obtainable in the form of hydrates, or their crystals may include, for example, the solvent used for crystallisation (present as solvates).

The present invention relates also to pharmaceutical preparations that contain a compound of formula (I) as active ingredient and that can be used especially in the treatment of the diseases mentioned above. Preparations for enteral administration, such as nasal, buccal, rectal or, especially, oral administration, and for parenteral administration, such as intravenous, intramuscular or subcutaneous administration, to warm-blooded animals, especially humans, are especially preferred. The preparations contain the active ingredient alone or, preferably, together with a pharmaceutically acceptable carrier. The dosage of the active ingredient depends upon the disease to be treated and upon the species, its age, weight, and individual condition, the individual pharmacokinetic data, and the mode of administration.

The invention relates also to pharmaceutical preparations for use in a method for the prophylactic or especially therapeutic treatment of the human or animal body, to a process for the preparation thereof (especially in the form of compositions for the treatment of tumours) and to a method of treating the above-mentioned diseases, primarily neoplastic diseases, especially those mentioned above.

The invention relates also to processes and to the use of compounds of formula (I) for the preparation of pharmaceutical preparations which contain compounds of formula (I) as active component (active ingredient).

Preference is given to a pharmaceutical composition that is suitable for administration to a warm-blooded animal, especially a human or commercially useful mammal, suffering from a disease that is responsive to the inhibition of microtubule depolymerisation, for example psoriasis or especially a neoplastic disease, comprising a correspondingly effective amount of a compound of formula I, or a pharmaceutically acceptable salt thereof when salt-forming groups are present, together with at least one pharmaceutically acceptable carrier.

A pharmaceutical composition for the prophylactic or especially therapeutic treatment of neoplastic and other proliferative diseases of a warm-blooded animal, especially a human or a commercially useful mammal requiring such treatment, especially suffering from such a disease, comprising a new compound of formula (I), or a pharmaceutically acceptable salt thereof, as active ingredient in a quantity that is prophylactically or especially therapeutically active against said diseases, is likewise preferred.

Pharmaceutical preparations contain from about 0.001 % to 95% of the active ingredient, whereby single-dose forms of administration preferably have from approximately 0.01% to 90% and multiple-dose forms of administration preferably have from approximately 0.01 % to 0.5% in the case of preparations for parenteral administration or 1% to 20% active ingredient in the case of preparations for enteral administration. Unit dose forms are, for example, coated and uncoated tablets, ampoules, vials, suppositories or capsules. Further dosage forms are, for example, ointments, creams, pastes, foams, tinctures, lipsticks, drops, sprays, dispersions, etc. Examples are capsules containing from about 0.002 g to about 1.0 g active ingredient.

The pharmaceutical preparations of the present invention are prepared in a manner known per se, for example by means of conventional mixing, granulating, coating, dissolving or lyophilising processes.

Preference is given to the use of solutions of the active ingredient, and also suspensions or dispersions, especially isotonic aqueous solutions, dispersions or suspensions which, for example in the case of lyophilised preparations which contain the active ingredient on its own or together with a carrier, for example mannitol, can be made up before use. The pharmaceutical preparations may be sterilised and/or may contain excipients, for example preservatives, stabilisers, wetting agents and/or emulsifiers, solubilisers, salts for regulating the osmotic pressure and/or buffers and are prepared in a manner known per se, for example by means of conventional dissolving or lyophilising processes. The said solutions or suspensions may contain viscosity-increasing agents, typically sodium carboxymethylcellulose, carboxymethylcellulose, dextran, polyvinylpyrrolidone, or gelatin, or also solubilisers, for example Tween™ 80.

Suspensions in oil contain as the oil component the vegetable, synthetic, or semisynthetic oils customary for injection purposes. In respect of such, special mention may be made of liquid fatty acid esters that contain as the acid component a long-chained fatty acid having from 8 to 22, especially from 12 to 22, carbon atoms, for example lauric acid, tridecylic acid, myristic acid, pentadecylic acid, palmitic acid, margaric acid, stearic acid, arachidic acid, behenic acid or corresponding unsaturated acids, for example oleic acid, elaidic acid, erucic acid, brassidic acid or linoleic acid, if desired with the addition of antioxidants, for example vitamin E, β-carotene or 3,5-di-tert-butyl-4-hydroxytoluene. The alcohol component of these fatty acid esters has a maximum of 6 carbon atoms and is a mono- or polyhydric, for example a mono-, di- or trihydric, alcohol, for example methanol, ethanol, propanol, butanol or pentanol or the isomers thereof, but especially glycol and glycerol. As fatty acid esters, therefore, the following are mentioned: ethyl oleate, isopropyl myristate, isopropyl palmitate, polyoxyethylene glycerol trioleate, unsaturated or saturated polyglycolised glycerides, and/or triglyceride of saturated fatty acids of chain length C₈ to C₁₂, but especially vegetable oils such as olive oil, cottonseed oil, almond oil, castor oil, sesame oil, soybean oil and more especially groundnut oil.

The manufacture of injectable preparations is usually carried out under sterile conditions, as is the filling, for example, into ampoules or vials, and the sealing of the containers.

Pharmaceutical compositions for oral administration can be obtained, for example, by combining the active ingredient with one or more solid carriers, if need be granulating a resulting mixture, and processing the mixture or granules, if desired, to form tablets or tablet cores, If need be by the inclusion of additional excipients.

Suitable carriers are especially fillers, such as sugars, for example lactose, saccharose, mannitol or sorbitol, cellulose preparations, and/or calcium phosphates, for example tricalcium phosphate or calcium hydrogen phosphate, and also binders, such as starches, for example corn, wheat, nice or potato starch, methylcellulose, hydroxypropyl methylcellulose, sodium carboxymethylcellulose, and/or polyvinylpyrrolidone, and/or, if desired, disintegrators, such as the above-mentioned starches, also carboxymethyl starch, crosslinked polyvinylpyrrolidone, alginic acid or a salt thereof, such as sodium alginate. Additional excipients are especially flow conditioners and lubricants, for example silicic acid, talc, stearic acid or salts thereof, such as magnesium or calcium stearate, and/or polyethylene glycol, or derivatives thereof.

Tablet cores may be provided with suitable, if need be enteric, coatings, using inter alia concentrated sugar solutions which may comprise gum arabic, talc, polyvinylpyrrolidone, polyethylene glycol and/or titanium dioxide, or coating solutions in suitable organic solvents or solvent mixtures, or, for the preparation of enteric coatings, solutions of suitable cellulose preparations, such as acetylcellulose phthalate or hydroxypropylmethylcellulose phthalate. Dyes or pigments may be added to the tablets or tablet coatings, for example for identification purposes or to indicate different doses of active ingredient.

Orally administrable pharmaceutical compositions also include hard capsules consisting of gelatin, and also soft, sealed capsules consisting of gelatin and a plasticiser, such as glycerol or sorbitol. The hard capsules may contain the active ingredient in the form of granules, for example in admixture with fillers, such as corn starch, binders, and/or glidants, such as talc or magnesium stearate, and if need be stabilisers. In soft capsules, the active ingredient is preferably dissolved or suspended in suitable liquid excipients, such as fatty oils, paraffin oil or liquid polyethylene glycols or fatty acid esters of ethylene or propylene glycol, to which stabilisers and detergents, for example of the polyoxyethylene sorbitan fatty acid ester type, may also be added.

Suitable rectally administrable pharmaceutical preparations are, for example, suppositories that consist of a combination of the active ingredient and a suppository base. Suitable suppository bases are, for example, natural or synthetic triglycerides, paraffin hydrocarbons, polyethylene glycols or higher alkanols.

The formulations suitable for parenteral administration are primarily aqueous solutions, for example in physiological saline, obtainable by diluting solutions in polyethylene glycol, of an active ingredient in water-soluble form, e.g. a water-soluble salt, or aqueous injectable suspensions containing viscosity-increasing agents, e.g. sodium carboxymethyl cellulose, sorbitol and/or dextran, and where appropriate stabilisers. The active ingredient, if need be together with excipients, can also be in the form of a lyophilisate and can be made into a solution before parenteral administration by the addition of suitable solvents. Solutions such as those used, for example, for parenteral administration can also be employed as infusion solutions.

Preferred preservatives are, for example, antioxidants, such as ascorbic acid, or microbicides, such as sorbic acid or benzoic acid.

The invention similarly relates to a process or a method for the treatment of one of the above-mentioned pathological conditions, especially a disease which responds to an inhibition of microtubule depolymerisation, especially a corresponding neoplastic disease. A compound of formula (I) can be administered as such or in the form of pharmaceutical compositions, prophylactically or therapeutically, preferably in an amount effective against the said diseases, to a warm-blooded animal, for example a human, requiring such treatment, the compounds especially being used in the form of pharmaceutical compositions. In the case of an individual having a bodyweight of about 70 kg the dose administered is from approximately 0.1 mg to approximately 1 g, preferably from approximately 0.5 mg to approximately 200 mg, of a compound of the present invention. Administration is preferably effected e.g. every 1 to 4 weeks, for example weekly, every two weeks, every three weeks or every 4 weeks.

The present invention also relates in particular to the use of a compound of formula (I), or a pharmaceutically acceptable salt thereof, especially a compound of formula (I) named as a preferred compound, or a pharmaceutically acceptable salt thereof, as such or in the form of a pharmaceutical formulation containing at least one pharmaceutically employable carrier, for the therapeutical and also prophylactic treatment of one or more of the above diseases.

The present invention also relates in particular to the use of a compound of formula (I), or a pharmaceutically acceptable salt thereof, especially a compound of formula I named as a preferred compound, or a pharmaceutically acceptable salt thereof, for the preparation of a pharmaceutical formulation for the therapeutical and also prophylactic treatment of one or more of the above diseases.

The following examples illustrate the invention, but are not intended to restrict their scope in any way.

### Example 1: Synthesis of compound 1

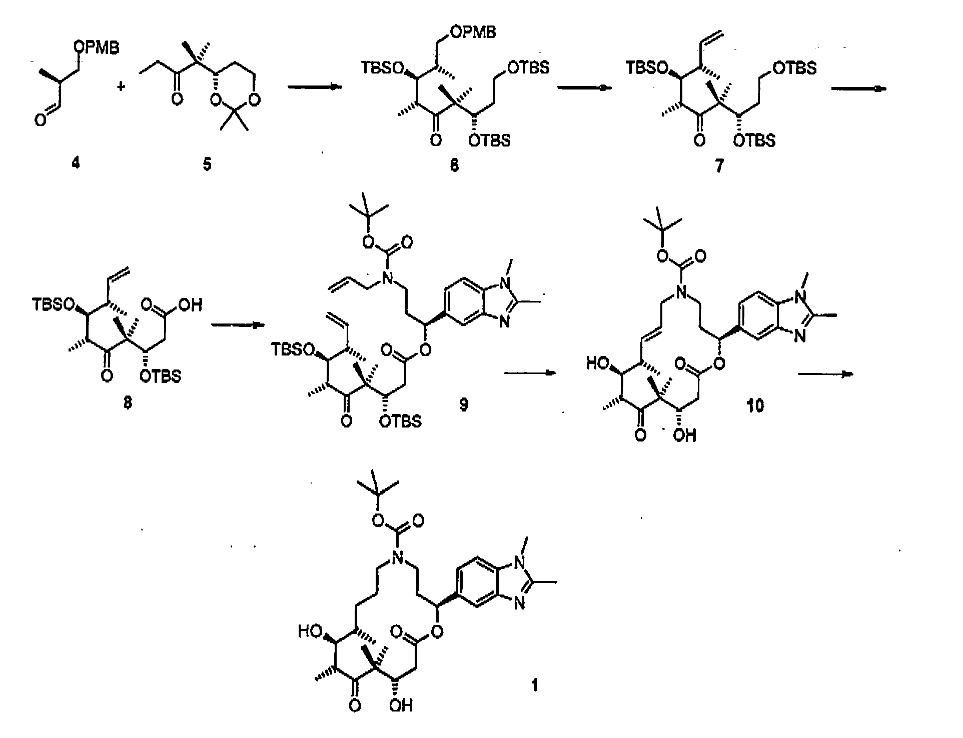

Compound **8** and its synthesis is described in J. Sun and S.C. Sinha, Angew. Chem. Int. Ed. 2002, 41, 1381-1383.

### (3S,6R,7S,8S)-((S)-3-(allyl(tert-butoxycarbonyl)amino)-1-(1,2-dimethyl-1H-benzo-[d]imidazol-5-yl)propyl)-3,7-bis(tert-butyldimethylsilyloxy)-4,4,6,8-tetramethyl-5-oxodec-9-enoate (9):

A mixture of acid **8** (190 mg), alcohol **11** (164 mg) and 4-(dimethylamino)pyridine (14 mg) in dichloromethane (2 ml) is treated at 0°C with dicyclohexylcarbodiimide (102 mg) in dichloromethane (0.5 ml). After stirring for 15 minutes at 0°C the reaction is allowed to stir for 15 hours at room temperature. The solution is absorbed on Celite^{®} and the product purified by flash chromatography (ethyl acetate/hexanes 5/3 → 2/1) to afford 193 mg of ester **9** as a colorless film. [α]²⁰_{D} =-17.2° (c = 0.55, CHCl₃); ¹H-NMR (400 MHz, CDCl₃) δ 7.58 (s, 1 H); 7.22-7.10 (m, 2H); 5.85 (m, 1 H); 5.76 (d, *J =* 7.0 Hz, 1 H); 5.67 (m, 1H); 5.05-4.90 (m, 4H); 4.30 (m, 1 H); 3.76-3.65 (m, 3H); 3.61 (s, 3H); 3.15 (s, br, 1 H); 2.99 (m, 2H); 2.51 (s, 3H); 2.40 (dd, *J*=13.6, 3.2 Hz, 1 H); 2.30 (dd, *J*=10.4, 6.5 Hz, 1 H); 2.18 (m, 1H); 2.05 (m, 2H); 1.38 (s, 9H); 1.11 (s, 3H); 0.99-0.90 (m, 9H); 0.87 (s, 9H), 0.84 (s, 9H); 0.04 (s, 3H); 0.00 (s, 3H), -0.02 (s, 3H); -0.05 (s, 3H); ¹³C-NMR (100 MHz, CDCl₃) δ 217.6, 171.1, 155.1, 152.1, 142.0, 139.8, 135.4, 134.0, 121.0 (br), 116.8, 116.3, 115.2, 108.7, 79.4, 76.1, 74.8, 73.7, 53.1, 49.8 (br), 45.9, 43.7, 43.3, 40.2, 35.1 (br), 29.7, 28.2, 26.1, 25.9, 24.8, 23.3, 19.7, 18.6, 18.3, 18.1, 15.0, 13.6, -3.7, -4.0, -4.3, -4.8; IR (film) 3020, 2930, 2854, 1710, 1362, 1215, 672; MS (MALDI) *m*/*z* (rel. intensity) 865 ([M+Na], 29), 843 ([M+H], 2), 523 (29), 342 (25), 286 (100), 143 (4); HR-MS (MALDI) (C₄₆H₇₉N₃O₇Si₂) calcd. 864.5349 (M+Na), found 864.5333 (M+Na).

(*S*)-*tert*-butyl *N*-allyl-*N*-(3-(1,2-dimethyl-1*H*-benzo[*d*]imidazol-5-yl)-3-hydroxy-propyl)-carbamate (**11**) was obtained in three steps from the acyl-sultam precursor **12** (K.-H. Altmann, G. Bold, G. Caravatti, A. Florsheimer, V. Guagnano, M. Wartmann, Bioorg. Med. Chem. Lett. 2000, 10, 2766-2768).

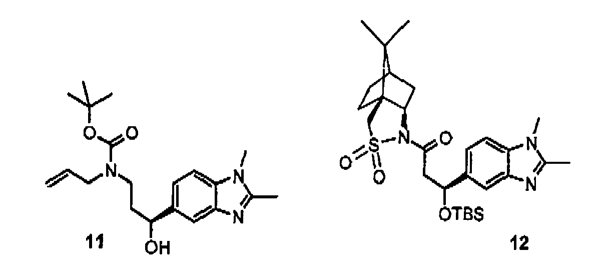

To a suspension of lithium aluminium hydride (0.3 g) in THF (20 ml) at 0°C is added the acylsultam precursor **12** (3.44 g) in THF (50 ml) dropwise with stirring. After the addition the mixture is allowed to stir for 1 hour at room temperature, before another portion of lithium aluminium hydride (0.1 g) is added. Stirring is continued for 1 hour, before water (2 ml) and 2N NaOH (3 ml) are added dropwise. After 15 minutes magnesium sulfate is added, and after another 15 minutes, the mixture is filtered. The solvent is removed under reduced pressure, and the residue is purified by flash chromatography (CHCl₃/MeOH/NEt₃ = 100/10/1) to furnish the corresponding diol, 5-(1(S),3-dihydroxypropyl)-1,2-dimethyl-1*H-*benzo[*d*]imidazol), (1.12 g) as a white solid. [α]²⁰_{D} = -11.6° (c = 0.64, MeOH). ¹H- and ¹³C-NMR data are as expected. IR (film) 3220, 2933, 2869, 2360, 2339, 1516, 1430, 1402, 1054, 804, 664; MS (MALDI) *m*/*z* (rel. intensity) 243 ([MNa⁺], 3), 221 ([MH⁺], 100), 189 (2), 111 (5), 96 (3); HR-MS (MALDI) (C₁₂H₁₆N₂O₂) calcd. 221.1290 (MH⁺), found 221.1285 (MH⁺).

The diol (1.10 g) together with 2,6-lutidine (2.95 ml) is dissolved in dichloromethane (30 ml) and treated dropwise at -78°C with a solution of TBSOTf (*tert*-butyl-dimethyl-silyloxy trifluoromethanesulfonate) (3.5 ml) in dichloromethane (10 ml). After 15 min at -78°C the reaction is stirred at room temperature for 1 hour, after which sat. NaHCO₃ solution (30 ml) and dichloromethane (30 ml) are added. The phases are separated, and the aqueous phase is extracted with dichloromethane (3 x 30 ml). Combined organic layers are dried over magnesium sulfate and the solvent is removed *in vacuo,* The resulting colourless oil (2.25 g) is dissolved in a 1/1 mixture of methanol/dichloromethane (50 ml) and camphersulfonic acid (0.98 g) is added at 0°C. After 1.5 hour another portion of camphersulfonic acid (0.60 g) is added and stirring is continued for 30 min. Work-up as described above and subsequent flash chromatography (CH₂Cl₂/MeOH = 20/1) yields the primary alcohol (1.24 g), wherein the benzylic hydroxy function is protected as the TBSO ether, as a colourless oil. [α]²⁰_{D} =-59.0° (c = 0.67, CHCl₃). ¹H- and ¹³C-NMR data are as expected. IR (film) 3222 (br), 2955, 2930, 2855, 2360, 2343, 1398, 1252, 836, 779, 668; MS (MALDI) *m*/*z* (rel. intensity) 357 ([MNa⁺], 6), 335 ([MH⁺], 100), 168 (6), 84 (2); HR-MS (MALDI) (C₁₈H₃₀N₂O₂Si) calcd 335.2155 (MH⁺), found 335.2149 (MH⁺).

This primary alcohol (1.15 g) is dissolved in a 3/1 mixture of diethyl ether and acetonitrile (29 ml), the solution is cooled to 0°C, and subsequently triphenylphosphine (1.35 g), imidazole (0.47 g) and iodine (1.31 g) are added. After 1 hour sat. sodium thiosulfate solution (2 ml), sat. NaHCO₃ (20 ml) and ethyl acetate (20 ml) are added. Work-up in the usual way (NaHCO₃/EtOAc) followed by flash chromatography (CH₂Cl₂/MeOH = 30/1) furnishes the corresponding iodide together with triphenylphosphine as a waxy solid, which is taken up in allylamine (15 ml) and heated to reflux for 20 hours. The solution is absorbed on Celite^{®} and the product purified by flash chromatography (CH₂Cl₂/MeOH = 20/1) to afford the amine (1.55 g) as a slightly yellow foam. The amine (0.78 g) in THF (20 ml) together with triethylamine (1.45 ml) is treated at 0°C with solid di-*tert*-butyl dicarbonate (1.36 g) and 4-dimethylamino-pyridine (0.02 g), and the mixture is allowed to stir for 3 hours at room temperature. Standard work-up (NaHCO₃/EtOAc) followed by flash chromatography (CH₂Cl₂/MeOH = 50/1) furnishes the BOC-protected amine (0.55 g) as a colourless oil. [α]^{2O}_{D} = -33.9° (c =1.00, CHCl₃). ¹H- and ¹³C-NMR data are as expected. IR (film) 2954, 2926, 2851, 1692, 1222, 840, 768; MS (MALDI) *m*/*z* (rel. intensity) 496 ([MNa⁺], 23), 286 (100), 173 (5), 143 (5); HR-MS (MALDI) (C₂₆H₄₃N₃O₃Si) calcd. 496.2966 (MNa⁺), found 496.2958 (MNa⁺).

In order to cleave the TBSO protecting group, this BOC-protected amine (0.49 g) in THF (30 ml) is treated with 1 M TBAF in THF (3.75 ml) at 0°C, and the mixture is stirred for **2** hours at room temperature. Standard work-up (NaHCO₃/EtOAc), followed by flash chromatography (CH₂Cl₂/MeOH = 25/1) leaves alcohol **11** (0.32 g) as a colourless oil. [α]²⁰_{D} = -7.0° (c = 1.27, CHCl₃). ¹H- and ¹³C-NMR data are as expected. IR (film) 3539 (br), 2969, 1713, 1427, 1362, 1227, 1094; MS (MALDI) *m*/*z* (rel. intensity) 382 ([MNa⁺], 9), 360 ([MH⁺], 3), 286 (100), 173 (6), 143 (7), HR-MS (MALDI) (C₂₀H₂₉N₃O₃) calcd 382.2101 (MNa⁺), found 382.2095 (MNa⁺).

### (2S,9S,10S,11R,14S,E)-tert-butyl-2-(1,2-dimethyl-1H-benzo[d]imidazol-5-yl)-10,14-dihydroxy-9,11,13,13-tetramethyl-12,16-dioxo-1-oxa-5-azacyclohexadec-7-ene-5-carboxylate (10):

Diene **9** (168 mg) is dissolved in dichloromethane (50 ml) and the resulting solution is treated with 2^{nd} generation Grubbs catalyst (tricyclohexylphosphine[1,3-bis(2,4,6-trimethylphenyl)-4,5-dihydroimidazol-2-ylidene][benzylidine]ruthenium(IV)dichloride) (20 mg). The mixture is heated to reflux for 2 hours before another portion of the catalyst (5 mg) is added. Stirring at reflux temperature is further continued for 2 hours, before another portion of catalyst (5 mg) is added. After stirring for 8 hours overall time Celite® is added and the solvent is evaporated under reduced pressure. Purification of the product by flash chromatography (ethyl acetate / hexanes = 5/3) furnishes 138 mg of the corresponding macrocycle as a white foam. [α]²⁰_{D} = -8.0° (c = 2.00, CHCl₃). ¹H- and ¹³C-NMR data are as expected. IR (film) 2978, 2930, 2855, 1745, 1695, 1373, 1244, 829, 775; MS (MALDI) *mlz* (rel. intensity) 837 ([M+Na], 43), 814 ([M+H], 6), 758 (100), 714 (6), 626 (13), 512 (6), 456 (7), 379 (4), 317 (7), 185 (9); HR-MS (MALDI) (C₄₄H₇₅N₃O₇Si₂) calcd. 836.5036 (M+Na), found 836.5022 (M+Na).

In a plastic vial the aforementioned macrocycle (30 mg) is dissolved in THF (3.5 ml) and pyridine (0.5 ml) is added. The mixture is cooled to 0°C, before HF*Pyridine (1.8 ml) is added dropwise by syringe. The reaction is stirred for 5 hours at room temperature, after which it is carefully added dropwise to an icecold saturated aqueous NaWCO₃ solution (100 ml). The aqueous phase is extracted with ethyl acetate (5 x 30 ml) and combined organic phases are dried over magnesium sulfate. The solvent is removed under reduced pressure to furnish 15 mg of alkene **10,** a colourless oil, which is directly used in the next step. Material of very high purity can be obtained by purification by HPLC (Symmetry^{®} C18 5 µm (19 x 100 mm; Waters); 25 ml/min of acetonitrile/H₂O, 0-2 min 5% acetonitrile → 5 min 70% acetonitrile → 11 min 70% acetonitrile, retention time 6.30 min); [α]²⁰_{D} = -113.0° (c = 1.47, CHC1₃). ¹H- and ¹³C-NMR data are as expected. IR (film) 3020, 2973, 1735, 1681, 1413, 1215, 775; MS (MALDI) *m*/*z* (rel. intensity) 608 ([M+Na)], 7), 586 ([M+H], 13), 530 (100), 486 (6), 442 (29), 342 (4), 265 (6), 185 (11), 173 (4); HR-MS (MALDI) (C₃₂H₄₉N₃O₇)calcd. 586.3487 (M+H), found 586.3477 (M+H).

### (2S,9S,10S,11R,14S)-tert-butyl-2-(1,2-dimethyl-1H-benzo[d]imidazol-5-yl)-10,14-dihydroxy-9,11,13,13-tetramethyl-12,16-dioxo-1-oxa-5-azacyclohexadecane-5-carboxylate (1):

Alkene **10** (15 mg, 26 µmol) is dissolved in dichloromethane (2 ml) and PADA (potassium azodicarboxylate, 300 mg) is added in one portion. The mixture is heated to reflux, before acetic acid (0.18 ml) is added dropwise over 15 minutes. Three more portions of PADA (300 mg) and acetic acid (0.18 ml) respectively are added, before the suspension is treated with aqueous NaHCO₃ (10 ml) and ethyl acetate (10 ml). The phases are separated and the aqueous phase is extracetd with ethyl acetate (2 x 30 ml). Combined organic phases are dried over magnesium sulfate and the solvent is removed *in vacuo.* The procedure described above is repeated twice and the resulting solid is purified by preparative HPLC (Symmetry^{®} C18 5 µm (19 x 100 mm; Waters); 25 ml/min of acetonitrile/H₂O, 0-2 min 20% acetonitrile → 9 min 90% acetonitrile, retention time: 7.05 min) to furnish 2.6 mg of pure **1** as a white solid together with 3.0 mg of a mixture of 1 (2.0 mg) and **10** (1.0 mg). [α]²⁰_{D} =-11.8° (c=1.20, CHCl₃); ¹H-NMR (500 MHz, DMSO-d₈, 318 K) δ 7.55 (s, 1 H); 7.40 (d, *J* = 8.3 Hz, 1H); 7.21 (d, *J* = 8.3 Hz, 1 H); 5.82 (m, 1H); 5.16 (d, *J* = 7.9 Hz, 1 H); 4.23 (d, *J* = 6.5 Hz, 1 H); 4.15 (s, br, 1 H); 3.71 (s, 3H); 3.57 (m, 1 H); 3.42-3.30 (m, 2H); 3.11-3.02 (m, 1H); 2.99-2.90 (s, br, 1 H); 2.55 (s, 3H); 2.49-2.41 (m, 1H); 2.40-2.32 (m, 1H); 2.12-2.00 (m, 3H); 1.60-1.50 (m, 1 H); 1.48-1.40 (m, 1 H); 1.45 (s, 9H); 1.40 (s, 3H); 1.22-1.10 (m, 2H); 1.04 (d, *J* = 6.7 Hz, 3H); 0.94 (s, 3H); 0.92 (d, *J* = 6.8 Hz, 3H); ¹³C-NMR (125 MHz, DMSO-d₆, 318 K) δ 217.8, 170.6, 158.7, 152.3, 142.0, 135.1, 133.4, 128.7, 119.2, 115.1, 77.9, 74.2, 73.7, 71.6, 54.9, 52.7, 47.0, 44.2, 42.4, 38.7, 36.1, 36.0, 29.5, 28.6, 28.0, 26.9, 20.2, 17.3, 14.3, 13.1; IR (film) 3460 (br), 3023, 2977, 2930, 2854, 1713, 1695, 1366, 1215, 750; MS (MALDI) *m*/*z* (rel. intensity) 610 ([M+Na)], 17), 588 ([M⁺], 4); HR-MS (MALDI) (C₃₂H₄₉N₃O₇) calcd. 610.3463 (M+Na), found 610.3452 (M+Na).

### Example 2: Alternative synthesis of compound 1

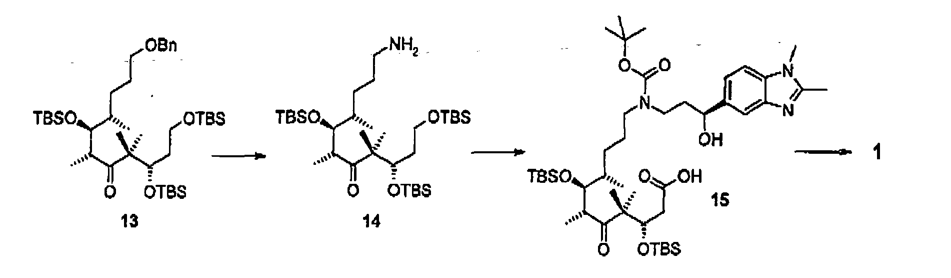

Compound **13** and its synthesis is described in K.-H. Altmann, G. Bold, G. Caravatti, D. Denni, A. Floersheimer, A. Schmidt, G. Rihs, M. Wartmann, Helv. Chim. Acta 2002, 85, 4086-4110.

### (5S,6R,9S)-5-((S)-5-aminonentan-2-yl)-9-(tert-butyldimethylsilyloxy)-2,2,3,3,6,8,8,13,13, 14,14-undecamethyl-4,12-dioxa-3,13-disilapentadecan-7-one (14):

To a solution of **13** (12.0 g) in ethyl acetate (250 ml) is added palladium on charcoal (10%, 2.0 g) and the mixture is stirred for 16 h under a hydrogen atmosphere. Filtration, evaporation of the solvent, and subsequent flash chromatography (ethyl acetate/hexane 1/20 → 1/8) furnishes 4.3 g of benzyl-deprotected alcohol and 6.5 g of unreacted starting material, which is subjected to the hydrogenation conditions described above to yield after 40 h another 4.7 g of the alcohol. [α]²⁰_{D} = -8.5° (c = 0.79, CHCl₃). ¹H- and ¹³C-NMR data are as expected. IR (film) 3387 (br), 2959, 2926, 2858,1692,1473,1255,1094, 986, 833, 768; MS (MALDI) *m*/*z* (rel. intensity) 655 ([MNa⁺], 18), 541 (26), 503 (23), 371 (6), 333 (100), 289 (13), 25910), 201 (5); HR-MS (MALDI) (C₃₃H₇₂O₅Si₃) calcd. 655.4580 (MNa⁺), found 655.4569 (MNa⁺).

The aforementioned alcohol (4.72 g) together with triphenylphosphine in THF (30 ml) at 0°C is treated dropwise with HN₃ in benzene (0.83 M, 18.10 ml) and diethylazodicarboxylate (1.76 ml) in THF (10 ml). Stirring is continued for 10 min at 0°C and 30 min at RT. Aqueous workup (NaHCO₃/ethyl acetate), drying over magnesium sulfate, evaporation of the solvent under reduced pressure and flash chromatography (hexane/ethyl acetate 10/1) of the residue affords the corresponding azide (4.69 g) as a colourless oil. [α]²⁰_{D} = -27.6° (c = 1.88, CHCl₃). ¹H- and ¹³C-NMR data are as expected. IR (film) 2952, 2934, 2855, 2089, 1692, 1469, 1252, 1094, 983, 836, 775; MS (MALDI) *m*/*z* (rel. intensity) 680 ([MH⁺)], 11), 607 (12), 525 (17), 503 (100), 331 (12), 285 (17), 263 (93), 241 (28); HR-MS (MALDI) (C₃₃H₇₁N₃O₄Si₃) calcd. 680.4645 (MH'), found 680.4632 (MH⁺).

The azide (4.62 g) is dissolved in ethanol (250 ml) together with palladium on charcoal (10%, 0.32 g) and the mixture is stirred for 3 h under a hydrogen atmosphere. Filtration, evaporation of the solvent and subsequent flash chromatography CH₂Cl₂/methanol/Et₃N 20/2/1) affords amine **14** (4.08 g) as a colourless oil. [α]²⁰_{D}= -31.7° (c = 0.81, CHCl₃). ¹H- and ¹³C-NMR data are as expected. IR (film) 2959, 2930, 2855, 1695, 1469, 1252, 1097, 836, 768; MS (MALDI) *m*/*z* (rel. intensity) 654 ([MNa⁺], 13), 632 ([MH⁺], 100), 503 (16), 500 (40), 368 (12), 289 (15), 263 (22), 230 (5), 158 (2); HR-MS (MALDI) (C₃₃H₇₃NO₄Si₃) calcd. 632.4920 (MH⁺), found 632.4910 (MH⁺).

### (3S,6R,7S,8S)-11-(tert-butoxycarbonyl((S)-3-(1,2-dimethyl-1H-benzo[d]imidazol-5-yl)-3-hydroxypropyl)amino)-3,7-bis(tert-butyldimethylsilyloxy)-4,4,6,8-tetramethyl-5-oxoundecanoic acid (15):

To a solution of amine **14** (4.05 g) in THF (150 ml) is added subsequently MS 4Å (~1g), aldehyde **12a** (2.30 g, synthesized analogously to the procedure described by K.-H. Altmann, G. Bold, G. Caravatti, A. Flörsheimer, V. Guagnano, M. Wartmann, Bioorg. Med. Chem Lett. 2000, 10, 2765-2768.), NaBH(OAc)₃ (2.17 g) and acetic acid (0.73 ml) and the resulting mixture is stirred for 2.5 h at room temperature. The reaction is cooled to 0°C and treated with sat. NH₄Cl solution (30 ml). Aqueous workup (NaHCO₃/ethyl acetate), drying over magnesium sulfate and evaporation of the solvent under reduced pressure furnishes a colourless oil (6.30 g), which is dissolved in THF (100 ml) and triethylamine (2.7 ml) is added. The mixture is cooled to 0°C and treated dropwise with a solution of Di-*tert-*butyl-dicarbonate (2.1 g) in THF (10 ml). Stirring is continued for 45 min at that temperature, before the reaction is worked up in the usual manner (NaHCO₃/ethyl acetate). Drying over magnesium sulfate, evaporation of the solvent under reduced pressure and flash chromatography (CH₂Cl₂/acetone 8/1) of the residue affords the BOC-protected amine (4.06 g) as a slightly yellow resin. [α]²⁰_{D} =-31.8° (c = 0.48, CHCl₃); ¹H-NMR (400 MHz, CDCl₃) δ 7.54 (s, 1H); 7.17-7.12 (m, 2H); 4.72 (m, 1H); 3.85 (m, 1 H); 3.71 (m, 1 H); 3.65-3.58 (m, 1 H), 3.61 (s, 3H); 3.57-3.50 (m, 1H); 3.24-2.88 (m, 5H); 2.51 (s, 3H); 1.97-1.70 (m, 2H); 1.55-1.39 (m, 4H); 1.36 (s, 9H), 1.35-1-12 (m, 8H), 1.10-0.90 (m, 7H); 0.89 (s, 9H); 0.87 (s, 9H), 0.85 (s, 9H) 0.82 (s, 9H); 0.05 (s, 3H); 0.03 (s, 3H), 0.02 (s, 3H), 0.00 (s, 3H), 0.00 (s, 3H) -0.02 (s, 3H); -0.03 (s, 3H); -0.27 (s, 3H); ¹³C-NMR (100 MHz, CDCl₃) δ 218.0,155.3,151.9 142.2,139.0,134.9,119.9, 116.1, 108.3, 78.7, 77.2, 73.7, 73,5, 60.8, 53.5, 47.5 (br), 45.0, 44.4, 39.6 (br), 38.5 (br), 38.0, 29.7, 28.3. 27.7, 26.7 (br), 26.1, 26.0, 25.8, 25.8, 24.4, 19.1, 18.4, 182, 18.1, 18.0, 17.5 (br), 15.0 (br), 13.6, -3.8, -3.8, -3.8, -3.8, -4.6, -5.1, -5.6, -5.7; IR (film) 2955, 2930, 2855, 1695, 1395, 1473, 1255, 1091, 833, 772; MS (MALDI) *m*/*z* (rel. intensity) 1071 ([MNa⁺], 30), 1049 ([MH⁺], 15), 917-(16), 861 (100), 596 (48), 503 (4), 464 (3), 331 (4), 185 (2); HR-MS (MALDI) (C₅₆H₁₀₉N₃O₇Si₄) calcd. 1070.7235 (MNa⁺), found 1070.7215 (MNa⁺).

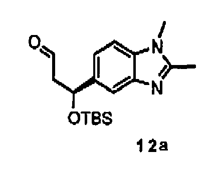

The resin (4.05 g) described above is dissolved in a 1/1 mixture of CH₂Cl₂/methanol (100 ml) at 0°C and camphersulfonic acid (0.94 g) is added. After stirring for 3 h at this temperature, sat. NaHCO₃ (100 ml) and dichloromethane (50 ml) are added, and the aqueous phase is extracted with dichloromethane (4 x 50 ml). Combined organic phases are dried over magnesium sulfate and the solvent is removed under reduced pressure. The resulting white foam (3.65 g) is dissolved in DMF (100 ml) together with pyridinium dichromate (21.7 g). After being stirred for 18 h sat. NaCl (100 ml) is added and the organic phase is extracted with ethyl acetate (8 x 50 ml). Combined organic phases are dried over magnesium sulfate, the solvent is removed under reduced pressure, and the residue is purified by flash chromatography (CH₂Cl₂/methanol 30/1 → 20/1) to yield 1.40 g of the corresponding acid. [α]²⁰_{D} = +1.6° (c = 1.17, CHCl₃). ¹H- and ¹³C-NMR data are as expected. IR (film) 2955, 292B, 2851, 1692, 1469, 1362, 1255, 1083, 836, 772; MS (MALDI) *m*/*z* (rel. intensity) 972 ([MNa⁺], 16), 949 ([MH⁺], 32); 817 (4), 760 (100), 628 (3), 331 (6), 263 (3); HR-MS (MALDI) (C₅₀H₉₃N₃O₃Si₃) calcd. 948.6343 (MH⁺), found 948.6324 (MH⁺).

The acid described above (1.33 g) in THF (30 ml) is treated with tetrabutylammonium fluoride (TBAF) in THF (1M, 8.00 ml) and the reaction mixture is allowed to stir for 20 h. Sat. NaHCO₃ (100 ml) and ethyl acetate (100 ml) are added, and the aqueous phase is repeatedly extracted with ethyl acetate (8 x 50 ml). Combined organic layers are dried over magnesium sulfate and the solvent is removed under reduced pressure to furnish compound 15 as a white foam (1.60 g), together with TBAF (ca. 10 %). It is used without further purification in the next step.

Seco acid 15 described above (1.60 g) is dissolved in THF and cooled to 0°C, before triethylamine (1.20 ml) and 2,4,6-trichlorobenzoylchloride (1.20 ml) are added dropwise. After 30 min at room temperature the mixture is diluted with toluene (200 ml) and transferred *via* cannula into a dropping funnel. This solution is slowly added to a solution of 4-(dimethylamino)pyridine (1.76 g) in toluene (900 ml) at 75°C over a period of 45 min. After the addition stirring is continued for 1 h at this temperature. The solvent is removed *in vacuo* and the residue purified by flash chromatography (ethyl acetate/hexane 2/1 → 5/1) to yield 372 mg of the macrocycle as a slightly yellow foam. [α]²⁰_{D} = -3.2° (c =1.90, CHCl₃). ¹H- and ¹³C-NMR data are as expected. IR (film) 2955, 2930, 2858, 1738, 1695, 1469, 1252, 990, 840, 775; MS (MALDI) *mlz* (rel. intensity) 839 ([MNa⁺], 19), 817 ([MH⁺], 8), 760 (100), 628 (4), 380 (4), 331 (17) ; HR-MS (MALDI) (C₄₄H₇₇N₃O₇Si₂) calcd 838.5192 (MNa⁺), found 838.5201 (MNa⁺).

To the aforementioned macrocycle (147 mg) together with pyridine (2.5 ml) in THF (17 ml) at 0°C is treated dropwise with HF*Pyridine (9 ml). The reaction is stirred for 2.5 h at room temperature, before it is carefully added dropwise to a vigorously stirred icecold saturated NaHCO₃ solution (200 ml). After adjusting the pH to 8.0 with 1M NaOH, the aqueous phase is extracted with ethyl acetate (5 x 50 ml) and combined organic phases are dried over magnesium sulfate. The solvent is removed under reduced pressure and the residue is purified by preparative HPLC (Symmetry^{®} C18 5 µm (19 x 100 mm; Waters); 25 ml/min of acetonitrile/H₂O, 0-2 min 5% acetonitrile → 5 min 70% acetonitrile → 11 min 70% acetonitrile, retention time 7.05 min) to furnish 42 mg of 1 as a white solid.

### Example 3: Synthesis of compounds 2 and 3

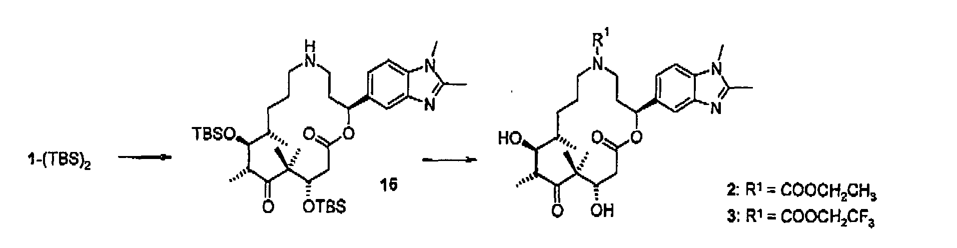

### (2S,9S,10S,11R,14S)-10,14-bis-(tert-butildimethylsilyloxy)-2-(1,2-dimethyl-1H-benzoi[d] imidazol-5-yl)-9,11,13,13-tetramethyl-1-oxa-5-azacyclohexadecane-12,16-dione (16):

A solution of **1**-(TBS)₂ (40 mg) in dichloromethane (3 ml) and anhydrous zinc bromide (50 mg) are stirred for 2.5 h at room temperature, after which diluted NaHCO₃ solution is added. The phases are separated and the aqueous phase is extracted with dichloromethane (5 x 5 ml). Combined organic phases are dried over magnesium sulfate and the solvent is removed *in vacuo.* The crude amine (37 mg) is essentially pure as checked by ¹H-NMR analysis and subsequently used in the next step.

### (2S,9S,10S,11R,14S)-ethyl-10,14-bis(tert-butyldimethylsilyloxy)-2-(1,2-dimethyl-1H-benzo[d]imidazol-5-yl)-9,11,13,13-tetramethyl-12,16-dioxo-1-oxa-5-azacyclohexa-decane-5-carboxylate (2-(TBS)₂):

Amine **16** (25.5 mg) is dissolved in THF (1.5 ml) and triethylamine (5 µl) is added. The mixture is cooled to 0°C and treated with ethyl chloroformate (4.3 mg). The reaction is allowed to stir for 1 h at room temperature and sat. NaHCO₃ solution is added. The phases are separated and the aqueous phase is extracted with ethyl acetate (3 x 10 ml). Combined organic phases are dried over magnesium sulfate and the solvent is removed *in vacuo.* Flash chromatography (dichloromethane/acetone 5/1 → 4/1) of the remaining solid furnishes the corresponding carbamate as a white foam (17 mg), which is directly deprotected in the next step.

### (2S,9S,11S,11R,14S)-ethyl-2-(1,2-dimethyl-1H-benzo[d]imidazol-5-yl)-10,14-dihydroxy-9,11,13,13-tetramethyl-12,16-dioxo-1-oxa-6-azacyclohexadecane-5-carboxylate (2)

In a plastic vial **2**-(TBS)₂ (17.0 mg) is dissolved in THF (1.7 ml) and pyridine (0.25 ml) is added. The mixture is cooled to 0°C, before HF*Pyridine (0.9 ml) is added dropwise over a period of 10 min. The reaction is stirred for 2 hours at room temperature, after which it is cooled to 0°C and another portion of HF*Pyridine (0.2 ml) is added. The reaction is allowed to stir for another 75 min at room temperature, before it is carefully added dropwise to a vigorously stirred icecold saturated NaHCO₃ solution (70 ml). The aqueous phase is extracted with ethyl acetate (3 x 20 ml) and combined organic phases are dried over magnesium sulfate. The solvent is removed under reduced pressure and the residue is purified by preparative HPLC (Symmetry^{®} C18 5 µm (19 x 100 mm; Waters); 25 ml/min of acetonitrile/H₂O, 0-2 min 5% acetonitrile → 5 min 70% acetonitrile → 10 min 70% acetonitrile, retention time 5.50 min) to yield **2** (6.6 mg) as a white powder. [α]²⁰_{D} = -8.3° (c = 0.50, CHCl₃); ¹H-NMR (500 MHz, DMSO-d₆, 318 K) δ 7.58 (s, 1H); 7.42 (d, *J* = 8.3 Hz, 1H); 7.23 (dd, *J =* 8.3, 1.3 Hz, 1H); 5.81 (dd, J = 8.4, 3.1 Hz, 1 H); 5.16 (d, *J* = 8.0 Hz, 1H); 4.23 (d, *J =* 6.5 Hz, 1H); 4.13 (m, 1H); 3.99 (q, J = 7.0 Hz, 2H); 3.71 (s, 3H); 3.57 (m, 1H); 3.52-3.33 (m, 2H); 3.29-3.16 (m, 1H); 3.15-3.07 (m, 1H); 2.99 (m, 1H); 2.53 (s, 3H); 2.47(dd, J = 14.4, 3.3 Hz, 1H); 2.37 (dd, J = 14.4, 10.4 Hz, 1H); 2.15-2.00 (m, 2H); 1.61-1.52 (m, 1H); 1.49-1.40 (m, 1 H); 1.39-1.23 (m, 2H); 1.30 (s, 3H); 1.22-1.15 (m, 1 H); 1.14 (t, J = 7.0 Hz, 3H); 1.03 (d, *J* = 6.7 Hz, 3H); 0.93 (s, 3H); 0.91 (d, *J* = 6.8 Hz, 3H); ¹³C-NMR (125 MHz, DMSO-d₆, 318 K) δ 218.1, 170.7, 155.3, 152.3, 142.0, 135-2, 133.7, 119.6,115.4,109.3, 74.3, 73.8, 72.0, 60.3. 52.9,47.2,44.5, 42.5, 38.8, 38.3, 35.8, 29.7, 27.0, 25.0, 20.5, 20.3, 17.5, 14.6, 14.3, 13.3; IR (film) 3359 (br), 2934, 2869, 1734, 1688, 1427, 1248, 1212, 1147, 1029, 754, 668; MS (MALDI) *mlz* (rel. intensity) 582 ([MNa⁺], 33), 560 ([MH⁺], 100), 472 (6), 303 (4), 263 (15), 241 (19), 185 (31); HR-MS (MALDI) (C₃₀H₄₅N₃O₇) calcd. 560.3330 (MH⁺), found 560.3321 (MH⁺).

### (2S,9S,10S,11R,14S)-2,2,2-trifluoroethyl-10,14-bis(tert-butyldimethylsilyloxy)-2-(1,2-dimethyl-1H-benzo[d]imidazol-5-yl)-9,11,13,13-tetramethyl-12,16-dioxo-1-oxa-5-azacyclohexa-decane-5-carboxylate (3-(TBS)₂).

Amine **16** (25.5 mg) is dissolved in THF (1.0 ml) and triethylamine (10 µl) is added. The mixture is cooled to 0°C and treated with a solution of 2,2,2-trifluoroethyl chloroformate in ethyl acetate (0.2 M, 210 µl). The reaction is allowed to stir for 30 min at 0°C and sat. NaHCO₃ solution is added. The phases are separated and the aqueous phase is extracted with ethyl acetate (3x10 ml). Combined organic phases are dried over magnesium sulfate and the solvent is removed *in vacuo.* Flash chromatography (dichloromethane/acetone 6/1 → 5/1) of the remaining solid furnishes the corresponding carbamate as a slightly yellow foam (24.6 mg), which is directly deprotected in the next step.

In a plastic vial **3**-(TBS)₂ (24.6 mg) is dissolved in THF (1.7 ml) and pyridine (0.3 ml) is added. The mixture is cooled to 0°C, before HF*Pyridine (1.0 ml) is added dropwise over a period of 10 min. The reaction is stirred for 3 hours at room temperature, before it is carefully added dropwise to a vigorously stirred icecold saturated NaHCO₃ solution (70 ml). The aqueous phase is extracted with ethyl acetate (3 x 20 ml) and combined organic phases are dried over magnesium sulfate. The solvent is removed under reduced pressure and the residue is purified by preparative HPLC (Symmetry^{®} C18 5 µm (19 x 100 mm; Waters); 12 ml/min of acetonitrile/H₂O, 50% acetonitrile, retention time 5.58 min) to yield **3** (7.0 mg) as a white powder. [α]²⁰_{D}= -3.2° (c = 0.58, CHCl₃); ¹H-NMR (500 MHz, DMSO-d₆) δ 7.58 (s, 1H); 7.42 (d, *J =* 8.3 Hz, 1H); 7.22 (m, 1H); 5.83 (d, *J =* 7.5 Hz, 1H); 5.29 (d, *J =* 8.5 Hz, 1H); 4.73-4.25 (m, 2H); 4.39 (d, *J =* 6.5 Hz, 1H); 4.16-4.00 (m, 1 H); 3.71 (s, 3H); 3.59-3.38 (m, 3H); 3.28-3.11 (m, 2H); 3.10-2.94 (m, 1 H); 2.52 (s, 3H); 2.49-2.30 (m, 2H); 2.20-1.95 (m, 2H); 1.68-1.51 (m, 1 H); 1.49-1.39 (m, 1H); 1.33 (s, 3H); 1.30-1.21 (m, 2H); 1.20-1.10 (m; 1H); 1.02 (d, *J =* 6.4 Hz, 3H); 0.92 (s, 3H); 0.88 (d, *J =* 8.1 Hz, 3H); ¹³C-NMR (125 MHz, DMSO-d₆, 318 K) δ 218.2, 170.8, 153.4, 152.6, 142.3, 135.4, 133.3, 123.7 (d, *J=* 278 Hz), 119.4, 115.6, 109.3, 74.5, 73.7, 72.3, 60.4 (q, *J* = 34.9 Hz), 52.8, 44.6, 40.6, 38.7, 36.3, 35.5, 29.7, 28.7, 26.9, 25.0, 20.7, 20.0, 17.5, 14.8, 13.4; IR (film) 3332 (br), 2933, 2851, 1717, 1430, 1287, 1276, 1165, 976, 757; MS (MALDI) *m*/*z* (rel. intensity) 636 ([MNa⁺], 14), 614 ([MH⁺], 100). 526 (11), 426 (11), 307 (6), 263 (8), 241 (5), 185 (20); HR-MS (MALDI) (C₃₀H₄₂F₃N₃O₇) calcd. 614.3048 (MH⁺), found 614.3037 (MH⁺).

### Example 4: Synthesis of compound 17

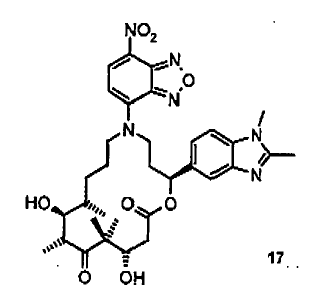

### (2S,9S,10S,11R,14S)-10,14-bis(tert-butyldimethylsilyloxy)-2-(1,2-dimethyl-1H-benzo[d]imidazol-5-yl)-9,11,13,13-tetramethyl-5-(7-nitrobenzo[c][1,2,5]oxadiazol-4-yl)-1-oxa-5-azacyclohexadecane-12,16-dione (17-(TBS)₂)

Amine 16 (18.5 mg) is dissolved in CH₂Cl₂ (1.5 ml) and triethylamine (3.6 µl) is added. The mixture is treated with 4-chloro-7-nitrobenzo-2,1,3-oxadiazole (10.3 mg) and subsequently heated to reflux for 2.5 h. After cooling to room temperature the solution is absorbed on Celite^{®} and the product is purified by flash chromatography (dichloromethanelacetone 2/1) to afford 13.5 mg of the title compound as bright red powder, which is directly deprotected in the next step.

### (2S,9S,10S,11R,14S)-2-(1,2-dimethyl-1H-benzo[d]imidazol-5-yl)-10,14-dihydroxy-9,11,13,13-tetramethyl-5-(7-nitrobenzo[c][1,2,5]oxadiazol-4-yl)-1-oxa-5-azacyclohexadecane-12,16-dione (17)

Following the procedure described for **3**, compound **17** is obtained from (**17**-(TBS)₂ (13.5 mg), THF(4 ml) and HF*Pyridine (1.5 ml), but without additional pyridine, after stirring for 3 h, subsequent work-up, and purification by HPLC (Symmetry^{®} C18 5 µm (19 x 100 mm; Waters); 25 ml/min of acetonitrile/H₂O, 0-2 min 5% acetonitrile → 5 min 60% acetonitrile → 11 min 60% acetonitrile, retention time 6.35 min) to yield 5.24 mg of a bright red powder, [α]²⁰_{D} = +565.1° (c = 0.24, CHCl₃); ¹H-NMR (400 MHz, CDCl₃) 6 8.37 (d, J = 9.0 Hz, 1 H); 7.68 (s, 1H); 7.23 (m, 2H); 6.09 (d, J = 9.1 Hz, 1H); 6.05 (d, J = 9.2 Hz, 1 H); 4.31 (m, 1H); 3.85 (br, m, 1H); 3.81 (m, 1 H); 3.72 (s, 3H); 3.67 (m, 1H); 3.45 (m, 1 H); 3.18-2.85 (m, 2H); 2.65-2.45 (m, 3H); 2.59 (s, 3H); 2.33-2.08 (m, 1H); 2.05-1.80 (m, 3H); 1.78-1.65 (m, 1H); 1.58-1.48 (m, 1H); 1.43 (s, 3H); 1.41-1.23 (m, 2H); 1.18 (d, J = 6.8 Hz, 3H); 1.07 (s, 3H); 1.05 (d, J = 7.0 Hz, 3H); ¹³C-NMR (100 MHz, CDCl₃)δ 220.8, 170.3, 152.8, 144.9, 144.8, 144.3, 142.3, 135.6, 135.4, 133.3, 122.2, 120.7, 115.6, 109.2, 100.8, 74.1, 73.1, 73.1, 53.4, 49.8 (br), 44.3 (br), 42.6, 38.8, 35-7,35.2 (br), 30.0, 29.7, 27.4, 20.2, 19.6, 16.6,13.8,12.4; IR (film) 3345 (br), 2955, 2926, 1735, 1556, 1283, 12fi2, 1219, 1147; MS (MALDI) *m*/*z* (rel. intensity) 651 ([MH⁺], 45), 503 (63), 463 (12), 331 (40), 285 (17), 263 (100), 241 (18), 131 (3); HR-MS (MALDI) (C₃₃H₄₂N₆O₈) calcd. 651.3137 (MH⁺), found 651.3125 (MH⁺).

### Example 5: Biological tests

### Preparation of pure αβ-tubulin (> 95%)

Tubulin was isolated as pure αβ-tubulin from fresh pig brain according to the method of Castoldi and Popov (Protein Expr. Purif. 2003, 32, 83-88). Fresh brains were obtained from the local slaughterhouse and processed immediately without prior cooling. 150-200 g cleaned pig brain was put into ice-cooled depolymerization buffer (50 mM MES [2-morpholinoethanesulphonic acid], 1 mM CaCl₂, adjusted to pH 6.9 with KOH) and homogenized in a Polytron mixer. The homogenate was centrifuged in a Sorvall SLA-1500 rotor at 14500 rpm for 60 min. The supernatant was transferred into an Erlenmeyer flask in high-molarity PIPES buffer (1 M PIPES [1,4-piperazinediethanesulfonic acid], 10 mM MgCl₂, 20 mM EGTA [ethylene glycol-bis-2(2-aminoethyl)-tetraacetic acid] adjusted to pH 6.9 with KOH) plus ATP (1.5 mM final concentration) and glycerol (98%) ad 300 ml. The resulting suspension was mixed and incubated at 37°C for 1 hour. Aliquots were transferred into ultracentrifuge tubes and centrifuged in a Beckman Ti50.2 rotor at.32500 rpm (96000 g) for 75 min at 30°C. The microtubule protein pellets were resuspended in depolymerization buffer and put on ice prior to ultracentrifugation at 4°C. The procedure was repeated for two polymerization cycles (total of 3 cycles) and the final αβ-tubulin pellets were resuspended in ice-cold Brinkley Buffer (BRB80; 80 mM PIPES, 1 mM MgCl₂, 1 mM EGTA adjusted to pH 6.8 with KOH) prior to shock-freezing in liquid nitrogen and subsequent storage at -80°C. αβ-tubulin purity and concentration were determined by SDS-PAGE gel electrophoresis and spectrophotometrically (*A* = *ε x c x d with* a given extinction coefficient of 115'000 M⁻¹cm⁻¹) at 280 nm. This procedure typically yielded 60-100 mg of αβ-tubulin per 100 g of brain.

### Turbidimetry analysis of polymer formation

Freshly thawed αβ-tubulin was centrifuged at 5000 g for 5 min at 5°C and then incubated with additional BRB80 buffer, drugs, DMSO vehicle, guanosine-5'-triphosphate (GTP) / glutamate (25 mM /2.7 M), respectively, in a 96-well plate in either 50 or 100 µl volumes. Experiments were either carried out in a 96-well quartz plate or in normal 96-well polystyrene plates (Falcon). The polymerization was monitored real-time at 340 nm in a temperature-controlled TECAN GeniosPro spectrophotometer. Depending on the specific experiment, the temperature was set either to room temperature (actual measuring temperature was 24-27°C) or 37°C. The concentration of DMSO was found to be highly critical, because concentrations >2% DMSO induced considerable microtubule formation. UV absorption of compounds was subtracted from absorption curves. All experiments, along with both negative (untreated αβ-tubulin) and vehicle controls, were carried out in triplicate. Experiments were performed at least with two different αβ-tubulin batches.

## Claims

1. A compound of formula (I) 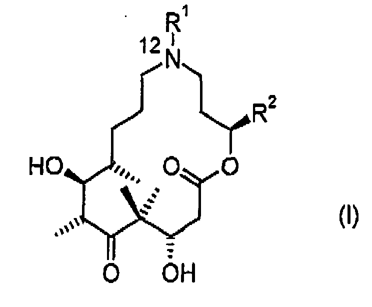 wherein R¹ is optionally substituted aminocarbonyl, optionally substituted alkoxycarbonyl, aryloxycarbonyl or heteroaryl and R² is a radical of formula (II) 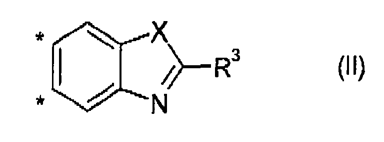 wherein X is S, O, NR⁴, -C(R⁵)=N-, -N=C(R⁶)- or -C(R⁵)=C(R⁶)-; R³ is hydrogen or optionally substituted lower alkyl, R⁴ is hydrogen, optionally substituted lower alkyl or aryl; R⁵ and R⁶ are, independently of one another, hydrogen or optionally substituted lower alkyl, and which is bound to the radical of formula (I) at one of the two carbon atoms indicated with an asterix, or salts thereof;

2. A compound of formula (I) according to claim 1, wherein
R¹ is aminocarbonyl, lower alkylaminocarbonyl, di-lower alkylaminocarbonyl, hydroxy-lower alkylaminocarbonyl, halogen-lower alkylaminocarbonyl, amino-lower alkylaminocarbonyl, lower alkylamino-lower alkylaminocarbonyl, di-lower alkylamino-lower alkylaminocarbonyl, carbamoyt-lower alkylaminocarbonyl; substituted aminocarbonyl wherein the substituents on nitrogen form a five, six or seven membered ring optionally containing oxygen, nitrogen or nitrogen substituted by lower alkyl; alkoxycarbonyl, hydroxy-lower alkoxycarbonyl, oxo-lower alkoxycarbonyl, lower alkoxy-lower alkoxycarbonyl, lower alkoxy-lower alkoxy-lower alkoxycarbonyl, halogen-lower alkoxycarbonyl, amino-lower alkoxycarbonyl, lower alkylamino-lower alkoxycarbonyl, di(lower alkyl)amino-lower alkoxycarbonyl, lower acylamino-lower alkoxycarbonyl, carboxy-lower alkoxycarbonyl, carbamoyl-lower alkoxycarbonyl, lower alkoxycarbonyl-lower alkoxycarbonyl, cyano-lower alkoxycarbonyl, phenyl-lower alkoxycarbonyl, di-hydroxy-lower alkoxycarbonyl, di-lower alkoxy-lower alkoxycarbonyl, di-halo-lower alkoxycarbonyl, tri-halo-lower alkoxycarbonyl; optionally substituted phenyloxycarbonyl, wherein the substituents are selected from amino; lower alkanoylamino, halogen, lower alkyl, halogen-lower alkyl, hydroxy; lower alkoxy, phenyl-lower alkoxy, nitro, cyano, C₈-C₁₂-alkoxy, carbamoyl, lower alkyl-carbamoyl, lower alkanoyl, phenyloxy, halogen-lower alkyloxy, lower alkoxycarbonyl, lower alkylmercapto, halogen-lower alkyl-mercapto, hydroxy-lower alkyl, lower alkanesulphonyl, halogen-lower alkane-sulphonyl, phenylsulphonyl, dihydroxyboranyl, 2-methyl-pyrimidin-4-yl, oxazol-5-yl, 2-methyl-1,3-dioxolan-2-yl, 1H-pyrazol-3-yl, 1-methyl-pyrazol-3-yl, and lower alkylenedioxy which is bonded to two adjacent carbon atoms; or pyrrolyl, thienyl, furyl, pyrazolyl, imidazolyl, triazolyl, oxazolyl, isoxazolyl, oxadiazolyl, thiazolyl, isothiazolyl, thiadiazolyl, pyridyl, pyridazinyl, pyrimidinyl, pyrazinyl, indolyl, benzimidazolyl, benzoxazolyl, benzoxadiazolyl, benzothiazolyl, benzothiadiazolyl, quinolinyl, isoquinolinyl, quinazolinyl, quinoxalinyl or purinyl, each optionally substituted by lower alkyl, halogen-lower alkyl, lower alkoxy-lower alkyl, lower alkoxy, lower alkoxy-lower alkoxy, amino optionally substituted by one or two substituents lower alkyl and one substituent lower alkylcarbonyl, halogen, or nitro; and
R² is a radical of formula (II), which is bound to the radical of formula (I) at one of the two carbon atoms indicated with an asterix, preferably by the carbon atom in para-position to X; wherein
R³ is hydrogen, lower alkyl, hydroxy-lower alkyl, halogen-lower alkyl, amino-lower alkyl, lower alkyl-amino-lower alkyl, di-lower alkyl-amino-lower alkyl or carbamoyl-lower alkyl;
X is S, O, NR⁴, -C(R⁵)=N-, -N=C(R⁶)- or -C(R⁵)=C(R⁶)-;
R⁴ is hydrogen, lower alkyl, hydroxy-lower alkyl, halogen-lower alkyl, amino-lower alkyl, lower alkyl-amino-lower alkyl, di-lower alkyl-amino-lower alkyl, carbamoyl-lower alkyl, or phenyl; and
R⁵ and R⁶ are, independently of one another, hydrogen, lower alkyl, hydroxy-lower alkyl, halogen-lower alkyl, amino-lower alkyl, lower alkyl-amino-lower alkyl, di-lower alkyl-amino-lower alkyl or carbamoyl-lower alkyl;
or salts thereof.

3. A compound of formula (I) according to claim 1, wherein
R¹ is aminocarbonyl, lower alkylaminocarbonyl, di-lower alkylaminocarbonyl, hydroxy-lower alkylaminocarbonyl, pyrrolidinocarbonyl, piperidinocarbonyl, N-methylpiperazinocarbonyl; morpholinocarbonyl; lower alkoxycarbonyl, hydroxy-lower alkoxycarbonyl, lower alkoxy-lower alkoxycarbonyl, lower alkoxy-lower alkoxy-lower alkoxycarbonyl, halogen-lower alkoxycarbonyl, phenyl-lower alkoxycarbonyl, di-hydroxy-lower alkoxycarbonyl, di-lower alkoxy-lower alkoxycarbonyl, di-halo-lower alkoxycarbonyl, tri-halo-lower alkoxycarbonyl; optionally substituted phenyloxycarbonyl, wherein the substituents are selected from amino; lower alkanoylamino, halogen, lower alkyl, halogen-lower alkyl, hydroxy; lower alkoxy, lower alkanoyl, and lower alkylenedioxy which is bonded to two adjacent carbon atoms; or pyrrolyl, thienyl, furyl, indolyl, benzimidazolyl, benzoxadiazolyl, benzothiazolyl, benzothiadiazolyl, pyridyl, pyrimidinyl or pyrazinyl, each optionally substituted by lower alkyl, halogen-lower alkyl, lower alkoxy-lower alkyl, lower alkoxy, lower alkoxy-lower alkoxy, amino optionally substituted by one or two substituents lower alkyl and one substituent lower alkylcarbonyl, halogen, or nitro; and
R² is a radical of formula (II), which is bound to the radical of formula (I) at one of the two carbon atoms indicated with an asterix, preferably by the carbon atom in para-position to X; wherein
R³ is hydrogen, lower alkyl, hydroxy-lower alkyl, halogen-lower alkyl, amino-lower alkyl, lower alkyl-amino-lower alkyl, di-lower alkyl-amino-lower alkyl or carbamoyl-lower alkyl;
X is S, O, NR⁴, -C(R⁵)=N-, -N=C(R⁵)- or -C(R⁵)=C(R⁶)-;
R⁴ is hydrogen, lower alkyl, hydroxy-lower alkyl, halogen-lower alkyl, amino-lower alkyl, lower alkyl-amino-lower alkyl, di-lower alkyl-amino-lower alkyl or carbamoyl-lower alkyl; and
R⁵ and R⁶ are, independently of one another, hydrogen, lower alkyl, hydroxy-lower alkyl, or halogen-lower alkyl;
or salts thereof.

4. A compound of formula (I) according to claim 1, wherein
R¹ is aminocarbonyl, lower alkylaminocarbonyl, di-lower alkylaminocarbonyl, hydroxy-lower alkylaminocarbonyl; lower alkoxycarbonyl, hydroxy-lower alkoxycarbonyl, lower alkoxy-lower alkoxycarbonyl, halogen-lower alkoxycarbonyl, phenyl-lower alkoxycarbonyl, di-halo-lower alkoxycarbonyl, tri-halo-lower alkoxycarbonyl, phenyloxycarbonyl; or benzoxadiazolyl or benzothiadiazolyl, each optionally substituted by lower alkyl, halogen-lower alkyl, halogen, or nitro; and
R² is a radical of formula (II), which is bound to the radical of formula (I) at one of the two carbon atoms indicated with an asterix, preferably by the carbon atom in para-position to X; wherein
R³ is hydrogen, lower alkyl, hydroxy-lower alkyl, halogen-lower alkyl, amino-lower alkyl, lower alkyl-amino-lower alkyl, di-lower alkyl-amino-lower alkyl or carbamoyl-lower alkyl;
X is S, O, NR⁴, -C(R⁵)=N-, -N=C(R⁵)- or -C(R⁵)=C(R⁶)-;
R⁴ is hydrogen, lower alkyl, hydroxy-lower alkyl, halogen-lower alkyl, amino-lower alkyl, lower alkyl-amino-lower alkyl, di-lower alkyl-amino-lower alkyl or carbamoyl-lower alkyl; and
R⁵ and R⁶ are, independently of one another, hydrogen, lower alkyl, hydroxy-lower alkyl, or halogen-lower alkyl;
or salts thereof.

5. A compound of formula (I) according to claim 1, wherein
R¹ is aminocarbonyl, lower alkylaminocarbonyl, di-lower alkylaminocarbonyl, hydroxy-lower alkylaminocarbonyl; lower alkoxycarbonyl, hydroxy-lower alkoxycarbonyl, lower alkoxy-lower alkoxycarbonyl, halogen-lower alkoxycarbonyl, phenyl-lower alkoxycarbonyl, di-halo-lower alkoxycarbonyl, tri-halo-lower alkoxycarbonyl, phenyloxycarbanyl; or benzoxadiazolyl or benzothiadiazolyl, each optionally substituted by lower alkyl, halogen-lower alkyl, halogen, or nitro; and
R² is a radical of formula (II), which is bound to the radical of formula (I) at one of the two carbon atoms indicated with an asterix, preferably by the carbon atom in para-position to X; wherein
R³ is hydrogen, methyl, ethyl, isopropyl, hydroxymethyl, 2-hydroxyethyl, aminomethyl, 2-aminoethyl, 2-dimethylaminoethyl, carbamoylmethyl, fluoromethyl, fluoroethyl, trifluoromethyl or 2,2,2-trifluoroethyl;
X is S, O NR⁴, wherein R⁴ is hydrogen, methyl, 2-hydroxyethyl, 2-aminoethyl, 2-dimethylaminoethyl or carbamoylmethyl; -C(R⁵)=N- or -N=C(R⁵)-, wherein R⁵ is hydrogen, methyl, hydroxymethyl, 2-hydroxyethyl, fluoromethyl, or trifluoromethyl; or - C(R⁵)=C(R⁶)-, wherein one of R⁵ or R⁶ is hydrogen and the other one is hydrogen, methyl, hydroxymethyl, 2-hydroxyethyl, fluoromethyl, or trifluoromethyl;
or salts thereof.

6. A compound of formula (I) according to claim 1, wherein
R¹ is lower alkoxycarbonyl, hydroxy-lower alkoxycarbonyl, lower alkoxy-lower alkoxycarbonyl, halogen-lower alkoxycarbonyl, phenyl-lower alkoxycarbonyl, di-halo-lower alkoxycarbonyl, tri-halo-lower alkoxycarbonyl, or benzoxadiazolyl optionally substituted by lower alkyl, halogen-lower alkyl, halogen, or nitro; and
R² is a radical of formula (II), which is bound to the radical of formula (I) at one of the two carbon atoms indicated with an asterix, preferably by the carbon atom in para-position to X; wherein
R³ is hydrogen, methyl, ethyl, hydroxymethyl, 2-hydroxyethyl, fluoromethyl, or trifluoromethyl;
X is S, O, NR⁴, wherein R⁴ is hydrogen, methyl or 2-hydroxyethyl; -C(R⁵)=N- or-N=C(R⁵)-, wherein R⁶ is hydrogen, methyl, hydroxymethyl, 2-hydroxyethyl, fluoromethyl, or trifluoromethyl; or -C(R⁵)=C(R⁶)-, wherein one of R⁵ or R⁶ is hydrogen and the other one is hydrogen, methyl, hydroxymethyl, 2-hydroxyethyl, fluoromethyl, or trifluoromethyl;
or salts thereof.

7. A compound of formula (I) according to claim 1, wherein
R¹ is lower alkoxycarbonyl, phenyl-lower alkoxycarbonyl, benzoxadiazolyl or nitro-benzoxadiazolyl; and
R² is a radical of formula (II), which is bound to the radical of formula (I) at one of the two carbon atoms indicated with an asterix, preferably by the carbon atom in para-position to X; wherein
R³ is hydrogen, methyl, ethyl, hydroxymethyl, 2-hydroxyethyl, fluoromethyl, or trifluoromethyl;
X is S, NR⁴, wherein R⁴ is hydrogen or methyl, or -C(R⁵)=C(R⁶)-, wherein R⁵ and R⁶ are hydrogen;
or salts thereof.

8. A compound of formula (I) according to claim 1, wherein
R¹ is tert-butoxycarbonyl, ethoxycarbonyl, 2,2,2-trifluoroethoxycarbonyl, or 7-nitro-4-benzoxadiazolyl; and R² is a radical of formula (IIIa), which is bound to the radical of formula (I) at the carbon atom indicated with an asterix.

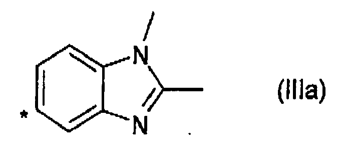

9. A method of manufacture of a compound of formula (I) according to claim 1, wherein
a) a diene of formula (IV) 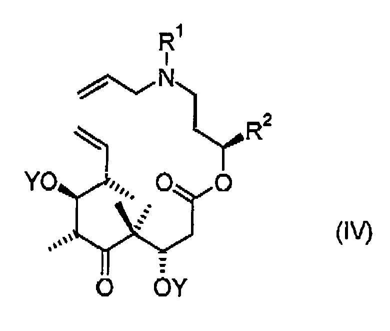 wherein R¹ and R² have the meanings given for compounds of formula (I), Y is a protecting group and wherein further functional groups that should not participate in the reaction are present if necessary in protected form, is cyclised in a metathesis reaction, the resulting double bond in position 9110 is hydrogenated, and protecting groups Y and any further protecting group are removed, or
b) a hydroxy-acid of formula M 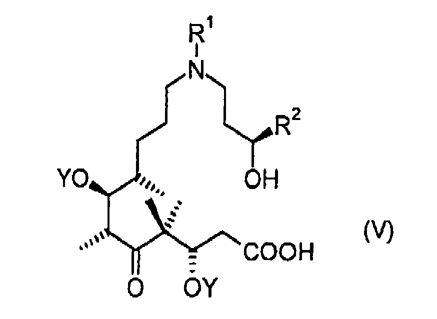
wherein R¹ and R² have the meanings given for compounds of formula (I), Y is a protecting group and wherein further functional groups that should not participate in the reaction are present if necessary in protected form, is cyclised, and protecting groups Y and any further protecting group are removed,
and, if desired, an obtainable compound of formula (I) is converted into a different compound of formula (I); an obtainable free compound of formula (I) is converted into a salt; an obtainable salt of a compound of formula (I) is converted into another salt or the free compound of formula (I); and/or obtainable isomeric mixtures of compounds of formula (I) are separated into the individual isomers.

10. The intermediate of formula (IV) 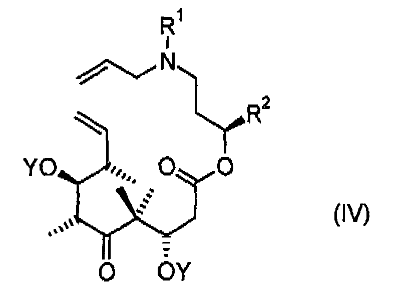 wherein R¹ and R² have the meanings as defined in claim 1 and Y is hydrogen or a protecting group.

11. The intermediate of formula M 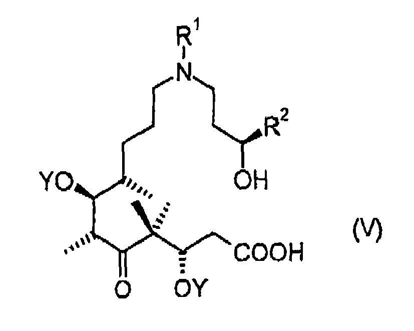 wherein R¹ and R² have the meanings as defined in claim 1 and Y is hydrogen or a protecting group.

12. A pharmaceutical preparation comprising a compound of formula (I) as defined in claim 1, or a salt thereof.

13. Use of a compound of formula (I) as defined in claim 1 for the manufacture of a pharmaceutical preparation for the treatment of proliferative diseases.

14. A method of treatment of a disease which responds to an inhibition of microtubule depolymerisation by administering a compound of formula (I) as claimed in claim 1 as such or in the form of pharmaceutical composition in an amount effective against the said disease to a warm-blooded animal requiring such treatment.
